# EUROPEAN PATENT SPECIFICATION

(11) **EP 2 369 015 B1**
(45) Date of publication and mention of the grant of the patent: **23.04.2014**
(21) Application number: 11157663.3
(22) Date of filing: 10.03.2011
(51) Int. Cl.: C12Q 1/68, G01N 33/53

(54) **Assay for localised detection of analytes**
Assay zur lokalisierten Erkennung von Analyten
Analyse pour la détection localisée d'analytes

(30) Priority: 15.03.2010 GB 201004292
(43) Date of publication of application: 28.09.2011
(73) Proprietor: Olink AB, 751 83 Uppsala (SE)
(72) Inventor: Fredriksson, Simon, 753 09, Uppsala (SE); Gullberg, Mats, 19146, Sollentun (SE)
(74) Representative: Dzieglewska, Hanna Eva

(56) References cited:
- EP-A1- 1 985 714
- WO-A2-2007/044903
- WO-A2-2009/012220
- SCHWEITZER B ET AL: "Immunoassays with rolling circle DNA amplification: a versatile platform for ultrasensitive antigen detection", PROCEEDINGS OF THE NATIONAL ACADEMY OF SCIENCES OF THE UNITED STATES (PNAS), NATIONAL ACADEMY OF SCIENCE, US, vol. 97, no. 18, 29 August 2000 (2000-08-29), pages 10113-10119, XP002207138, ISSN: 0027-8424, DOI: DOI:10.1073/PNAS.170237197
- GUSTAFSDOTTIR ET AL: "Proximity ligation assays for sensitive and specific protein analyses", ANALYTICAL BIOCHEMISTRY, ACADEMIC PRESS INC, NEW YORK, vol. 345, no. 1, 1 October 2005 (2005-10-01), pages 2-9, XP005078981, ISSN: 0003-2697, DOI: DOI:10.1016/J.AB.2005.01.018
- SCHALLMEINER EDITH ET AL: "SENSITIVE PROTEIN DETECTION VIA TRIPLE-BINDER PROXIMITY LIGATION ASSAYS", NATURE METHODS, NATURE PUBLISHING GROUP, GB, vol. 4, no. 2, 1 February 2007 (2007-02-01), pages 135-137, XP009084626, ISSN: 1548-7091, DOI: DOI:10.1038/NMETH974
- SÖDERBERG OLA ET AL: "Characterizing proteins and their interactions in cells and tissues using the in situ proximity ligation assay.", METHODS (SAN DIEGO, CALIF.) JUL 2008 LNKD- PUBMED:18620061, vol. 45, no. 3, July 2008 (2008-07), pages 227-232, XP002638191, ISSN: 1095-9130
- JOERGER R D ET AL: "ANALYTE DETECTION WITH DNA-LABELED ANTIBODIES AND POLYMERASE CHAIN REACTION", CLINICAL CHEMISTRY, AMERICAN ASSOCIATION FOR CLINICAL CHEMISTRY, WASHINGTON, DC, vol. 41, no. 9, 1 September 1995 (1995-09-01), pages 1371-1377, XP001018334, ISSN: 0009-9147

## Description

The present invention relates to a method for detecting an analyte in a sample. More particularly, the invention relates to localised detection of an analyte, especially detection of an analyte *in situ.* The method relies on the principle of so-called "proximity probing", wherein the analyte is detected by the binding of two probes, which when brought into proximity by binding to the analyte (hence "proximity" probes) allow a signal to be generated. In the new method of the invention, the signal is generated by a rolling circle amplification reaction (RCA) mediated, directly or indirectly, by an enzyme carried by one of the proximity probes and requiring a nucleic acid moiety which is carried on the other probe. The production of the RCA product allows a signal to be generated which is localised to the analyte. Advantageously, this allows an analyte to be detected *in situ.* Also provided are kits for performing such an assay.

It is generally desirable to be able sensitively, specifically and quantitatively to detect the presence of an analyte in a sample, including for example in fixed or fresh cells or tissues. The concept of proximity probing, i.e. requiring the binding of multiple probes to an analyte in proximity in order for a signal to be produced, has been developed in recent years and many assays based on this principle are now well known in the art. For example, so-called Proximity ligation assays (PLAs), rely on proximal binding of proximity probes to an analyte to generate a signal from a ligation reaction; the proximity probes may carry oligonucleotides (referred to as "oligonucleotide arms"), which when brought into proximity by binding of the probes to the analyte, may be ligated together and a signal may be generated from the ligation product, for example by detecting the ligation product using a nucleic acid amplification reaction. Such an assay in described for example in WO 97/00446. Alternatively, rather than being ligated to each other, the oligonucleotide arms of the proximity probes when in proximity may template the ligation of one or more added oligonucleotides to each other, including an intramolecular ligation to circularise an added linear oligonucleotide, for example based on the so-called padlock probe principle, wherein analogously to a padlock probe, the ends of the added linear oligonucleotide are brought into juxtaposition for ligation by hybridising to a template, here an oligonucleotide arm of the proximity probe (in the case of a padlock probe the target nucleic acid for the probe). Various such assay formats are described in WO 01/61037. Proximity ligation assays may be performed on a solid phase (e.g. when the analyte is immobilised or captured on a solid surface) for example as described in WO 97/00446, or in solution (so-called homogenous assays) as described in WO 01/61037.

In WO 99/49079 an embodiment of a proximity ligation assay is described involving a pair of proximity probes, wherein the oligonucleotide arms of the probes, which are attached to the analyte-specific binding moieties of the respective probes, have complementarity to the 5' and 3' ends, and a region between said ends, respectively, of an added linear oligonucleotide (akin to a "padlock probe"). When both probes of the proximity probe pair are brought into proximity due to binding to the same analyte, the oligonucleotide arms of the respective probes are able to hybridise to the respective parts of the added oligonucleotide. The proximity probe arm with complementarity to the 5' and 3' ends of the added oligonucleotide templates the juxtaposed hybridisation, and ligation (on addition of an appropriate ligase), of said ends, resulting in circularisation of the added oligonucleotide. This circularised oligonucleotide is then detected by rolling circle amplification (RCA) using the other probe arm as primer; the free end of the "templating" arm is only a 5' end, and hence it cannot serve as a primer for extension by a polymerase. This function is performed by the oligonucleotide arm of the other probe of the pair, which is hybridised to a region of the added oligonucleotide between the ligated ends, and has a free 3' end. Upon the addition of an appropriate polymerase, the presence of analyte in the sample may be detected by a rolling circle amplification (RCA) of the circularised oligonucleotide. The concatemeric RCA product, which can only be formed when the proximity probes bind in proximity, provides a "surrogate" marker for detection of the analyte. It will be appreciated that the single added oligonucleotide of this disclosure can be replaced by two oligonucleotides which may be ligated together to form a circle (such ligation may be templated by one or both probe arms, but one of the probe arms will have a free 3' end to act as a primer).

Such an embodiment has been developed as the "Duolink^{R}" assay, commercially provided by Olink AB, Uppsala, Sweden, for *in situ* detection of proteins. See for example Söderberg et a/. Nature Methods 3(12), 2006, 995-1000.

Not all proximity assays are based on ligation. WO 2007/044903 discloses proximity probe-based assays for detecting analytes in solution (i.e. not immobilised or *in situ*), which rely on the formation and detection of a released nucleic acid cleavage product. Some of the described embodiments involve a probe comprised of an analyte-binding moiety and an attached enzyme, which enzyme acts on a nucleic acid moiety attached to the analyte-binding moiety of a second probe, resulting in the release of a detectable nucleic acid cleavage product. The release of a detectable nucleic acid cleavage product is a defining feature of WO 2007/044903 concept and is therefore common to all of the disclosed embodiments. By its nature, such a "released" cleavage product does not remain attached to the analyte-probe complex, and becomes free in solution. There is furthermore no disclosure in WO 2007/044903 of an embodiment wherein the analyte is immobilised on a solid phase or in a native context such as in cells or tissues. Hence, the assays of WO 2007/044903 cannot facilitate localised detection of an analyte in a sample.

Analyte detection assays, including in some embodiments proximity probe-like reagents, wherein a polymerase enzyme attached to an analyte-binding moiety of one probe acts on a nucleic acid moiety attached to the analyte-binding moiety of a second probe, are described in WO 2009/012220. In these assays, the action of the "tethered" polymerase which is part of one of the probes of a probe pair results in the generation of a template, free in solution, which is susceptible to amplification by an added polymerase. Unlike the tethered polymerase, the added polymerase is only able to act on the template generated by the tethered polymerase, and not directly on the nucleic acid moiety of the non-polymerase-containing probe of the probe pair. The action of the added polymerase results in amplification of the generated template, the amplified copies being detectable and indicative of the presence of analyte in the sample, according to the proximity probing principle. However, like WO 2007/044903, the assays of WO 2009/012220 result in detectable nucleic acid molecules free in solution (i.e. not immobilised) and therefore cannot facilitate localised detection of an analyte in a sample.

The present inventors have sought further to develop a proximity-based assay which can be used for detection of analytes *in situ,* that is in the native context in which they occur i.e. in cells or tissues. In this regard, it has been found that localised detection of an analyte in a sample may be achieved by designing proximity probes such that one of the probes of the probe pair carries an enzyme moiety which acts, directly or indirectly, to facilitate RCA primed from a nucleic acid moiety carried by the other probe and templated by a circular nucleic acid molecule hybridised thereto.

In more detail, in such a method the sample is contacted with at least one proximity probe pair wherein each probe of the pair can bind to the analyte simultaneously with the binding to the analyte of the other probe of the pair. One probe of the pair of proximity probes comprises an enzyme moiety attached to the part of the probe having binding affinity for the analyte. The enzyme moiety is capable of directly or indirectly facilitating or enabling (e.g. mediating) rolling circle amplification (RCA) which may advantageously be primed from a nucleic acid moiety attached to the analyte-binding part of the other probe of the pair. The RCA is templated by a circularised molecule hybridised to said nucleic acid moiety. The resulting RCA product is connected to (e.g. continuous with) the said nucleic acid moiety and as a result is attached to one of the analyte-bound probes. The RCA product is therefore effectively localised to the vicinity of the analyte in the sample. The concatemeric nature of the RCA product enables qualitative or quantitative detection by a variety of convenient means, including microscopic visual detection of labelled hybridisation probes. Hence, the present invention enables detection of the analyte *in situ.*

The new method of the present invention therefore provides a new and not previously contemplated alternative means for achieving sensitive, specific, quantitative and localised detection of an analyte in a sample, whereby simple and convenient visualisation means, such as microscopy, may be used. The new assay method of the invention also may allow for improved (i.e. increased) resolution, for example as compared to proximity assays using nucleic acid-only based proximity probes. As nucleic acid may in some cases be "sticky" (i.e. bind non-specifically) to sample components, the presence of a nucleic acid moiety only on one of the probes of the pair may provide the advantage of reducing background signal relative to conventional proximity ligation assay methods in which both probes of the pair contain nucleic acid moieties. Further advantages may be associated with particular embodiments of the method of the invention, as discussed further below.

Accordingly, the present invention provides a method for localised *in situ* detection of an analyte in a sample, comprising:
(a) contacting said sample with at least one set of at least first and second proximity probes, wherein said probes each comprise an analyte-binding moiety and can simultaneously bind to the analyte, and wherein
   (i) said first proximity probe comprises a nucleic acid moiety attached at one end to the analyte-binding moiety, wherein a circular or circularisable oligonucleotide is hybridised to said nucleic acid moiety before, during or after said contacting step; and
   (ii) said second proximity probe comprises an enzyme moiety, attached to the analyte-binding moiety, capable of directly or indirectly enabling rolling circle amplification (RCA) of the circular or, when it is circularised, of the circularisable oligonucleotide hybridised to the nucleic acid moiety of the first proximity probe, wherein said RCA is primed by said nucleic acid moiety of said first proximity probe;
(b) if necessary, circularising said oligonucleotide, to produce a circularised template for RCA;
(c) subjecting said circular or circularised template to RCA, wherein if the enzyme moiety of the second proximity probe in step (a)(ii) is a DNA polymerase, this step does not utilise a free DNA polymerase and wherein the nucleic acid moiety of the first proximity probe directly or indirectly mediates the priming of the RCA reaction;
(d) detecting a product of said RCA.

In the method, it is advantageous that the nucleic acid moiety of the first proximity probe directly or indirectly mediates the priming of the RCA reaction in step (c). If the nucleic acid moiety is attached to the analyte binding moiety via its 5' end, then the 3' end will be free to prime such a reaction. Thus, in one embodiment of the method above in step (a)(i) the nucleic acid moiety is attached at its 5' end, and said RCA (e.g. as referred to in step (a)(ii)) is primed by said nucleic acid moiety of said first proximity probe. Thus, in such an embodiment, it will be understood that in step (c) the RCA is primed by the nucleic acid moiety of said first proximity probe. As an alternative to the nucleic acid moiety directly mediating the priming of the RCA (by acting as a primer), the nucleic acid moiety may indirectly mediate the priming. In such an embodiment, for example, the nucleic acid moiety may be attached at its 3' end to the analyte binding moiety. A "primer oligonucleotide" may be hybridised to the nucleic acid moiety to provide a free 3' end to act as primer for the RCA. This may be hybridised before, during or after the contacting step.

Howsoever the primer is provided, the RCA is templated by the circular or circularisable oligonucleotide which is hybridised to the nucleic acid moiety of the first proximity probe. The enzyme moiety of the second proximity probe is required in order for the RCA to occur. Thus, the enzyme moiety directly or indirectly enables, or in other words facilitates, the RCA.

The method of the invention depends upon detecting the presence of an analyte in a sample by detecting the interaction between two or more proximity probes, when such probes are bound to the analyte. The interaction between the probes is thus proximity-dependent; the binding of the probes, together, on the analyte brings them into proximity, such that they (or more particularly, their respective nucleic acid moiety and enzyme moiety) may interact. Accordingly, by detecting the interaction (which may lead directly or indirectly to the RCA reaction), i.e. by detecting the resulting RCA product, the analyte may be detected.

The term "detection" is used broadly herein to include any means of determining, or measuring (e.g. quantitatively determining), the presence of the analyte (i.e. if, or to what extent, it is present, or not) in the sample. "Localised" detection means that the signal giving rise to the detection of the analyte is localised to the analyte. The analyte may therefore be detected in or at its location in the sample. In other words the spatial position (or localisation) of the analyte within the sample may be determined (or "detected"). Thus "localised detection" may include determining, measuring, assessing or assaying the presence or amount and location, or absence, of analyte in any way. Quantitative and qualitative determinations, measurements or assessments are included, including semi-quantitative. Such determinations, measurements or assessments may be relative, for example when two or more different analytes in a sample are being detected, or absolute. Absolute quantification may be accomplished by inclusion of known concentration(s) of one or more control analytes and/or referencing the detected level of the target analyte with known control analytes (e.g., through generation of a standard curve). Alternatively, relative quantification can be accomplished by comparison of detected levels or amounts between two or more different target analytes to provide a relative quantification of each of the two or more different analytes, i.e. relative to each other.

As used herein, the term "*in situ*" refers to the detection of an analyte in its native context, i.e. in the place or situation in which it normally occurs. Thus, this may refer to the natural or native localisation of an analyte. In other words, the analyte may be detected where, or as, it occurs in its native environment or situation. Thus, the analyte is not moved from its normal location i.e. it is not isolated or purified in any way, or transferred to another location or medium etc. Typically, this term refers to the analyte as it occurs within a cell or tissue, e.g. its native localisation within the cell or tissue and/or within its normal or native cellular or tissue environment.

The "analyte" may be any substance (e.g. molecule) or entity it is desired to detect by the method of the invention. The analyte is the "target" of the assay method of the invention. The analyte may accordingly be any biomolecule or chemical compound it may be desired to detect, for example a proteinaceous molecule, e.g. a peptide or protein, or nucleic acid molecule or a small molecule, including organic and inorganic molecules. The analyte may be a cell or a microorganism, including a virus, or a fragment or product thereof. It will be seen therefore that the analyte can be any substance or entity for which a specific binding partner (e.g. an affinity binding partner) can be developed. All that is required is that the analyte is capable of simultaneously binding two binding partners (more particularly, the analyte-binding domains of at least two proximity probes). By "simultaneously" in this context is meant that the analyte, which may be a single molecule or a molecular complex or aggregate, is capable of being bound by at least two proximity probes at the same time; it does not imply that the respective binding events must occur synchronously.

Proximity probe-based assays, such as that of the present invention, have found particular utility in the detection of proteins or polypeptides. Analytes of particular interest may thus include proteinaceous molecules such as peptides, polypeptides, proteins or prions or any molecule which includes a protein or polypeptide component, etc., or fragments thereof. The analyte may be a single molecule or a complex that contains two or more molecular subunits, which may or may not be covalently bound to one another, and which may be the same or different. Thus in addition to cells or microorganisms, such a complex analyte may also be a protein complex. Such a complex may thus be a homo- or hetero-multimer. Aggregates of molecules e.g. proteins may also be target analytes, for example aggregates of the same protein or different proteins. The analyte may also be a complex between proteins or peptides and nucleic acid molecules such as DNA or RNA. Of particular interest may be the interactions between proteins and nucleic acids, e.g. regulatory factors, such as transcription factors, and DNA or RNA. Thus, an "analyte" according to the present invention may include the components of an interaction, for example a cellular interaction e.g. an interaction between proteins, or between other cellular components. Thus, in the case of an interaction between two (or more) molecules or entities, the analyte may be the two (or more) such molecules or entities when they have interacted. The assay method of the invention may therefore be advantageously used to detect such an interaction, including both the presence or the absence (e.g. disruption) of the interaction. An analyte may also be a modifed form of a protein or other molecule, for example a phosphorylated protein. This may be detected for example by employing as one of the proximity probe pair a proximity probe in which the analyte-binding moiety binds to the modified form of the analyte, for example to the site of phosphorylation.

By "sample" is meant any sample in which an analyte may occur, to the extent that such a sample is amenable to localised *in situ* detection. Typically, the sample may be any biological, clinical and environmental samples in which the analyte may occur, and particularly a sample in which the analyte is present at a fixed, detectable or visualisable position in the sample. The sample will thus be any sample which reflects the normal or native ("*in situ*") localisation of the analyte, i.e any sample in which it normally or natively occurs. Such a sample will advantageously be a cell or tissue sample. Particularly preferred are samples such as cultured or harvested or biopsied cell or tissue samples in which the analyte may be detected to reveal the localisation of the analyte relative to other features of the sample. As well as cell or tissue preparations, such samples may also include, for example, dehydrated or fixed biological fluids, and nuclear material such as chromosome/chromatin preparations, e.g. on microscope slides. The samples may be freshly prepared or they may be prior-treated in any convenient way such as by fixation or freezing. Accordingly, fresh, frozen or fixed cells or tissues may be used, e.g. FFPE tissue (Formalin Fixed Paraffin Embedded).

Thus, subject to the requirement that the analyte is present at a fixed, detectable or visualisable position in the sample, representative samples include any material which may contain a biomolecule, or any other desired or target analyte, including for example foods and allied products, clinical and environmental samples etc. The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells, plant cells, algae including blue-green algae, fungi, bacteria, protozoa etc. Representative samples thus include clinical samples, e.g. whole blood and blood-derived products such as plasma, serum and buffy coat, blood cells, urine, faeces, cerebrospinal fluid or any other body fluids (e.g. respiratory secretions, saliva, milk, etc), tissues, biopsies, as well as other samples such as cell cultures, cell suspensions, conditioned media or other samples of cell culture constituents, etc.

As stated in step (a) above, the sample is contacted with at least one set of proximity probes. The use of a single set of proximity probes occurs in the case of a "simplex" (as opposed to "multiplex") embodiment of the method of the invention, i.e. when a single analyte is to be detected. It will be understood that the term "single" as used in relation to a set of proximity probes, or the analyte, means single in the sense of a "single species" i.e. a plurality of analyte molecules of the same type may be present in the sample for detection, and a plurality of identical sets of proximity probes specific for that analyte may be used, but such pluralities relate only to a single (species of) analyte or proximity probe set. In multiplex embodiments a sample is assayed for two or more different (species of) target analytes. In such embodiments, the sample is contacted with a set of proximity probes (i.e. optionally, and most commonly, a plurality of identical sets per analyte species) for each (species of) target analyte, such that the number of (species of, i.e. non-identical) sets contacted with the sample may be two or more, e.g., three or more, four or more etc. Typically, up to 10, 15 or 20 probe sets may be used. Such methods find particular use in high-throughput applications.

The proximity probes for use in the method of the invention comprise an analyte-binding moiety, and a nucleic acid moiety ("first" proximity probe) or enzyme moiety ("second" proximity probe), and are in effect detection probes which bind to the analyte (via the analyte-binding moiety), the binding of which may be detected (to detect the analyte) by means of detecting the interaction which occurs between the respective nucleic acid and enzyme moieties thereof upon such binding. Accordingly the probes may be viewed as affinity ligands or binding partners for the analyte. The nucleic acid moiety of the first probe and the enzyme moiety of the second probe are coupled to the analyte-binding moiety of the respective probes and this "coupling" or connection may be by any means known in the art, and which may be desired or convenient, and may be direct or indirect e.g. via a linking group. For example, the moieties may be associated with one another by covalent linkage (e.g. chemical cross-linking) or by non-covalent association e.g. via streptavidin-biotin based coupling (biotin being provided on one domain and streptavidin on the other). Many procedures for effecting such coupling or conjugation are known in the art. For example, oligonucleotides may be attached to antibodies using sulpho-SMCC chemistry (Pierce Biotechnology) which links primary amines on antibodies with thiol on an oligonucleotide forming a thio-ester covalent bond (Soderberg et al, 2006, Nature Methods, 3(12), 995-1000). These reagents may additionally be purified from excess oligonucleotides using standard size-exclusion chromatography. Methods for coupling enzymes to antibodies are also well known in the art, as described for example in Hashida et al (1984), J. Appl. Biochem. 6, 56-63; Imagawa et al (1982), J. Appl. Biochem. 4, 41-57; O'Sullivan et al (1979), Anal. Biochem. 100, 100-108.

The "analyte-binding moiety" may be any binding partner for the target analyte, and it may be a direct or indirect binding partner therefor. Thus it may bind to the target analyte directly, or indirectly via an intermediary molecule or binding partner which binds to the target analyte, the analyte-binding moiety binding to said intermediary molecule (binding partner). Particularly, the analyte-binding moiety or the intermediary binding partner is a specific binding partner for the analyte. Hence, any reference to the probes of the method of the invention binding to an analyte encompasses both direct probe binding, and indirect probe binding via an intermediary molecule which itself directly binds to the analyte. A binding partner is any molecule or entity capable of binding to its target, e.g. target analyte, and a specific binding partner is one which is capable of binding specifically to its target (e.g. the target analyte), namely that the binding partner binds to the target (e.g. analyte) with greater affinity and/or specificity than to other components in the sample. Thus binding to the target analyte may be distinguished from non-target analytes; the specific binding partner either does not bind to non-target analytes or does so negligibly or non-detectably or any such non-specific binding, if it occurs, may be distinguished. The binding between the target analyte and its binding partner is typically non-covalent.

The analyte-binding moiety may be selected to have a high binding affinity for a target analyte. By high binding affinity is meant a binding affinity of at least about 10⁻⁴ M, usually at least about 10⁻⁶ M or higher, e.g., 10⁻⁹ M or higher. The analyte-binding moiety may be any of a variety of different types of molecules, so long as it exhibits the requisite binding affinity for the target analyte when present as part of the proximity probe. In other embodiments, the analyte-binding moiety may be a ligand that has medium or even low affinity for its target analyte, e.g., less than about 10⁻⁴ M.

The analyte-binding moiety may be a small molecule or large molecule ligand. By small molecule ligand is meant a ligand ranging in size from about 50 to about 10,000 daltons, usually from about 50 to about 5,000 daltons and more usually from about 100 to about 1000 daltons. By large molecule is meant a ligand ranging in size from about 10,000 daltons or greater in molecular weight.

The small molecule may be any molecule, as well as a binding portion or fragment thereof, that is capable of binding with the requisite affinity to the target analyte. Generally, the small molecule is a small organic molecule that is capable of binding to the target analyte. The small molecule will include one or more functional groups necessary for structural interaction with the target analyte, e.g. groups necessary for hydrophobic, hydrophilic, electrostatic or even covalent interactions. Where the target analyte is a protein, the small molecule ligand will include functional groups necessary for structural interaction with proteins, such as hydrogen bonding, hydrophobic-hydrophobic interactions, electrostatic interactions, etc., and will typically include at least an amine, amide, sulfhydryl, carbonyl, hydroxyl or carboxyl group, preferably at least two of the functional chemical groups. The small molecule may also comprise a region that may be modified and/or participate in covalent linkage to the nucleic acid moiety or enzyme moiety (as appropriate) of the proximity probe, without substantially adversely affecting the small molecule's ability to bind to its target analyte.

Small molecule affinity ligands often comprise cyclical carbon or heterocyclic structures and/or aromatic or polyaromatic structures substituted with one or more of the above functional groups. Also of interest as small molecules are structures found among biomolecules, including peptides, saccharides, fatty acids, steroids, purines, pyrimidines, derivatives, structural analogues or combinations thereof. Such compounds may be screened to identify those of interest, and a variety of different screening protocols are known in the art.

The small molecule may be derived from a naturally occurring or synthetic compound that may be obtained from a wide variety of sources, including libraries of synthetic or natural compounds. For example, numerous means are available for random and directed synthesis of a wide variety of organic compounds and biomolecules, including the preparation of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known small molecules may be subjected to directed or random chemical modifications, such as acylation, alkylation, esterification, amidification, etc, to produce structural analogues.

As such, the small molecule may be obtained from a library of naturally occurring or synthetic molecules, including a library of compounds produced through combinatorial means, i.e. a compound diversity combinatorial library. When obtained from such libraries, the small molecule employed will have demonstrated some desirable affinity for the target analyte in a convenient binding affinity assay. Combinatorial libraries, as well as methods for their production and screening, are known in the art.

The analyte-binding moiety may also be a large molecule. Of particular interest as large molecule analyte-binding moieties are antibodies, as well as binding fragments and derivatives or mimetics thereof. Where antibodies are the analyte-binding moieties, they may be derived from polyclonal compositions, such that a heterogeneous population of antibodies differing by specificity are each "tagged" with the same nucleic acid or enzyme moiety, or monoclonal compositions, in which a homogeneous population of identical antibodies that have the same specificity for the target analyte are each tagged with the same nucleic acid or enzyme moiety. As such, the analyte-binding moiety may be either a monoclonal or polyclonal antibody. In yet other embodiments, the analyte-binding moiety is an antibody binding fragment or derivative or mimetic thereof, where these fragments, derivatives and mimetics have the requisite binding affinity for the target analyte. For example, antibody fragments, such as Fv, F(ab)₂ and Fab may be prepared by cleavage of the intact protein, e.g. by protease or chemical cleavage. Also of interest are recombinantly- or synthetically-produced antibody fragments or derivatives, such as single chain antibodies or scFvs, or other antibody derivatives such as chimeric antibodies or CDR-grafted antibodies, where such recombinantly- or synthetically-produced antibody fragments retain the binding characteristics of the above antibodies. Such antibody fragments or derivatives generally include at least the V_{H} and V_{L} domains of the subject antibodies, so as to retain the binding characteristics of the subject antibodies. The above-described antibodies, fragments, derivatives and mimetics thereof may be obtained from commercial sources and/or prepared using any convenient technology, where methods of producing polyclonal antibodies, monoclonal antibodies, fragments, derivatives and mimetics thereof, including recombinant derivatives thereof, are well known to those of skill in the art.

Also suitable for use as analyte-binding moieties are polynucleic acid aptamers. Polynucleic acid aptamers may be RNA oligonucleotides which may act to selectively bind proteins, much in the same manner as a receptor or antibody (Conrad et al., Methods Enzymol. (1996), 267(Combinatorial Chemistry), 336-367). In certain embodiments where the analyte-binding moiety is a nucleic acid, e.g., an aptamer, the target analyte is not a nucleic acid.

Importantly, the analyte-binding moiety will be one that can be attached to the nucleic acid or enzyme moiety (as appropriate) without substantially abolishing the binding affinity of the analyte-binding moiety to its target analyte.

In addition to the types of analyte-binding moieties discussed above, the analyte-binding moiety may also be a lectin, a soluble cell-surface receptor or derivative thereof, an affibody or any combinatorially-derived protein or peptide from phage display or ribosome display or any type of combinatorial peptide or protein library. Combinations of any analyte-binding moiety may be used.

The binding sites on the analyte for the respective analyte-binding moieties of the proximity probes in a set may be the same or different. Thus, for example in the case of a homomeric protein complex or aggregate comprising two or more identical subunits or protein constituents, the analyte-binding moieties of two or more probes in a set may be the same. Where the analyte is a single molecule or comprises different sub-units or constituents (e.g. a heteromeric complex or an aggregate of different proteins, or the components of an interaction), the analyte-binding moieties of the probes in a set will be different.

The length of the nucleic acid moiety of the first proximity probe can be constructed to span varying molecular distances. Similarly, the molecular distance between the enzyme moiety of the second proximity probe and the analyte-binding moiety to which it is attached may be variably pre-determined. For example the enzyme moiety may be attached by means of a linker, which may be of variable length. Hence, binding sites on the analyte for the analyte-binding moieties need not be on the same molecule. They may be on separate, but closely positioned, molecules. For example, the multiple binding domains of an organism, such as a bacteria or cell, or a virus, or the components of an interaction, can be targeted by the methods of the present invention.

As noted above, the analyte-binding moiety may bind to the analyte directly or indirectly. In the case of indirect binding, the target analyte may first be bound by a specific binding partner (or affinity ligand), and the analyte-binding moiety of the proximity probe may bind to the specific binding partner. This enables the design of proximity probes as universal reagents. For example the analyte-specific binding partner may be an antibody, and a universal proximity probe set may be used to detect different analytes by binding to the Fc regions of the various different analyte-specific antibodies. If the analyte-binding moieties of a proximity probe set are antibodies and the analyte-specific binding partners are also antibodies, the former may be secondary antibodies whilst the latter may be primary antibodies.

As mentioned above, the nucleic acid and enzyme moieties of, respectively, the first and second proximity probes interact to form, or to participate in, or contribute to, the formation of, a detectable product (RCA product), which may be detected to report the presence or amount, and location, of the analyte. These moieties may thus be regarded as reactive functionalities which interact to provide, or to participate in the provision of, the signal by means of which the analyte is detected (more particularly, to form a signal-giving product). When two or more analytes are present in the same sample they may be detected simultaneously using two or more sets of proximity probes, each set of proximity probes being designed to form on interaction a unique "analyte-specific" RCA product. These unique RCA products may be detected and quantified separately or in parallel using methods well known in the literature including liquid chromatography, electrophoresis, mass spectrometry, DNA array technology, multi-colour real-time PCR, flow cytometry and microscopic visualisation of labelled, e.g. fluorescently-labelled, probes hybridised to the concatemeric RCA product. The RCA product may also be labelled using enzymes or enzyme substrates, to lead to an enzymically-generated signal e.g. with horse radish peroxidase (HRP) or alkaline phosphatase (AP).

The "nucleic acid moiety" of the first proximity probe may be a single-stranded nucleic acid molecule (e.g. an oligonucleotide) or a partially double-stranded and partially single-stranded molecule, providing that a portion, e.g. a terminal portion at the 3' or 5' end (i.e. an end not connected to the analyte-binding moiety), of the nucleic acid moiety is sufficiently single-stranded to permit hybridisation to the circular or circularisable (i.e. linear) oligonucleotide of step (a)(i) above. In particular embodiments the nucleic acid moiety is made up of a single-stranded nucleic acid. As mentioned above, in one embodiment step (a)(i) of the method above, the nucleic acid moiety is connected or conjugated via its 5' end to the analyte-binding moiety of the probe in order that its 3' end may, after enzymatic "unblocking" if necessary (discussed below), be used to prime the RCA in step (b) of the method. In alternative embodiments the primer for RCA may be separately provided as a primer oligonucleotide which hybridises to a 3' end attached nucleic acid moiety. Thus, in step (a)(i) above, the nucleic acid moiety is connected or conjugated via one end thereof to the analyte-binding moiety, in order that the other end directly or indirectly provides a primer for the RCA. In embodiments in which the analyte-binding moiety is or comprises a nucleic acid, reference to the nucleic acid moiety means the part of the probe which does not have specificity for the analyte, and which contains the portion (e.g. a terminal portion) complementary to a portion of the circular or circularisable oligonucleotide.

The nucleic acid moiety is generally of a length sufficient to allow a productive hybridisation with the circular or circularisable oligonucleotide of step (a)(i). By "productive hybridisation" is meant the formation of a duplex of sufficient length and integrity to support RCA using the nucleic acid molecule as template (if necessary, after ligation (circularisation) and optionally the incorporation of (oligo)nucleotides, as discussed below). Optionally, the RCA may use the 3' end of the nucleic acid moiety as a primer.

Alternatively, a primer may also be hybridised to the nucleic acid moiety. Accordingly, the length of the nucleic acid moiety may need to accommodate hybridisation of a primer oligonucleotide.

Hence, the nucleic acid moiety contains a portion (e.g. a 3' terminal portion) of sequence complementary to a portion of sequence of said circular or circularisable oligonucleotide, said portion of the nucleic acid moiety being of a length sufficient to allow a productive hybridisation. This does not require, but does include, 100% complementarity between said portion of the nucleic acid moiety and the portion of the circular or circularisable oligonucleotide. Thus "complementary", as used herein, means "functionally complementary", i.e. a level of complementarity sufficient to mediate a productive hybridisation, which encompasses degrees of complementarity less than 100%. Thus, the region of complementarity between the nucleic acid moiety and the portion of the circular or circularisable oligonucleotide is at least 5 nucleotides in length and is preferably 10 or more nucleotides in length, e.g. 6, 7, 8, 9, 11, 12, 13, 14, 15, 16 ,17, 18, 19, 20, 21, 22, 23, 24, 25, 26, 27, 28, 29, 30, 35, 40, 45, 50 etc nucleotides. It may for example be up to 30, 40, 50, 60, 70, 80, 90 or 100 nucleotides in length.

As discussed below, in embodiments wherein the oligonucleotide of step (a)(i) above is not yet circularised (i.e. is circularisable, i.e. linear), said portion (e.g. 3' terminal portion) of the nucleic acid moiety must be functionally complementary to the sequence at the respective ends of the oligonucletide. In some such embodiments, the ends of the circularisable oligonucleotide may be complementary to non-contiguous parts of the portion of the nucleic acid moiety, i.e. said portion of the nucleic acid moiety may consist of oligonucleotide-complementary parts separated by a part which is not complementary to said oligonucleotide. Hence, reference to said portion of the nucleic acid moiety being complementary to a portion of the circularised or circularisable oligonucleotide encompasses, *inter alia,* a portion comprising complementary parts separated by a non-complementary part (which latter part will have complementarity to (oligo)nucleotides incorporated between the ends of the circularisable oligonucleotide), providing that said portion of the nucleic acid moiety can undergo a productive hybridisation with the nucleic acid molecule and (oligo)nucleotides incorporated therein.

Further to this length requirement of the nucleic acid moiety, i.e. that it must be capable of a productive hybridisation with the circular or circularisable oligonucleotide (and optionally with a primer oligonucleotide), as discussed above the nucleic acid moiety may be of a length suitable to span a particular molecular distance as dictated by the nature of the analyte and the position thereon of the respective binding sites for the analyte-binding moieties. It will be understood that an appropriate length of nucleic acid moiety is related to the distance between the enzyme moiety and analyte-binding moiety of the second probe, and the flexibility of the connection between the two moieties of said second probe. Those skilled in the art will appreciate that the nucleic acid and enzyme moieties of the respective probes of a probe set should be designed in concert in situations where this appears necessary, e.g. when the respective binding sites on the analyte are, relatively, "far apart".

The nucleic acid moiety may be from about 8 nucleotides to about 1000 nucleotides in length, and in certain embodiments may range from about 8 to about 500 nucleotides in length including from about 8 to about 250 nucleotides in length, e.g., from about 8 to about 160 nucleotides in length, such as from about 12 to about 150 nucleotides in length, from about 14 to about 130 nucleotides in length, from about 16 to about 110 nucleotides in length, from about 8 to about 90 nucleotides in length, from about 12 to about 80 nucleotides in length, from about 14 to about 75 nucleotides in length, from about 16 to about 70 nucleotides in length, from about 16 to about 60 nucleotides in length, and so on. In certain representative embodiments, the nucleic acid moiety may range in length from about 10 to about 80 nucleotides in length, from about 12 to about 75 nucleotides in length, from about 14 to about 70 nucleotides in length, from about 34 to about 60 nucleotides in length, and any length between the stated ranges. In some embodiments, the nucleic acid moiety is usually not more than about 28, 29, 30, 31, 32, 33, 34, 35, 36, 37, 38, 39, 40, 41, 42, 44, 46, 50, 55, 60, 65, or 70 nucleotides in length.

The nucleic acid moiety may be made up of ribonucleotides and/or deoxyribonucleotides as well as synthetic nucleotide residues that are capable of participating in Watson-Crick-type or analogous base pair interactions. Thus, the nucleic acid domain may be DNA or RNA or any modification thereof e.g. PNA or other derivatives containing non-nucleotide backbones. Locked nucleic acids (LNA) may be used.

The sequence of the nucleic acid moiety of the first proximity probe is chosen with respect to the sequence of at least a portion of the circular or circularisable oligonucleotide. Thus, the sequence is not critical as long as a portion (e.g. a 3' terminal portion) may undergo a productive hybridisation with a portion of the circular or circularisable oligonucleotide. Once the sequence is selected or identified, the nucleic acid moiety may be synthesized using any convenient method.

The two moieties of the first proximity probe may be joined together either directly through a bond or indirectly through a linking group. Where linking groups are employed, such groups may be chosen to provide for covalent attachment of the nucleic acid and analyte-binding moieties through the linking group, as well as maintain the desired binding affinity of the analyte-binding moiety for its target analyte. Linking groups of interest may vary widely depending on the analyte-binding moiety. The linking group, when present, is in many embodiments biologically inert. A variety of linking groups are known to those of skill in the art and find use in the subject proximity probes. In representative embodiments, the linking group is generally at least about 50 daltons, usually at least about 100 daltons and may be as large as 1000 daltons or larger, for example up to 1000000 daltons if the linking group contains a spacer, but generally will not exceed about 500 daltons and usually will not exceed about 300 daltons. Generally, such linkers will comprise a spacer group terminated at either end with a reactive functionality capable of covalently bonding to the nucleic acid or analyte-binding moieties. Spacer groups of interest may include aliphatic and unsaturated hydrocarbon chains, spacers containing heteroatoms such as oxygen (ethers such as polyethylene glycol) or nitrogen (polyamines), peptides, carbohydrates, cyclic or acyclic systems that may possibly contain heteroatoms. Spacer groups may also be comprised of ligands that bind to metals such that the presence of a metal ion coordinates two or more ligands to form a complex. Specific spacer elements include: 1,4-diaminohexane, xylylenediamine, terephthalic acid, 3,6-dioxaoctanedioic acid, ethylenediamine-N,N-diacetic acid, 1,1'-ethylenebis(5-oxo-3-pyrrolidinecarboxylic acid), 4,4'-ethylenedipiperidine. Potential reactive functionalities include nucleophilic functional groups (amines, alcohols, thiols, hydrazides), electrophilic functional groups (aldehydes, esters, vinyl ketones, epoxides, isocyanates, maleimides), functional groups capable of cycloaddition reactions, forming disulfide bonds, or binding to metals. Specific examples include primary and secondary amines, hydroxamic acids, N-hydroxysuccinimidyl esters, N-hydroxysuccinimidyl carbonates, oxycarbonylimidazoles, nitrophenylesters, trifluoroethyl esters, glycidyl ethers, vinylsulfones, and maleimides. Specific linker groups that may find use in the subject proximity probes include heterofunctional compounds, such as azidobenzoyl hydrazide, N-[4-(p-azidosalicylamino)butyl]-3'-[2'-pyridyldithio]propionamid), bis-sulfosuccinimidyl suberate, dimethyladipimidate, disuccinimidyltartrate, N-maleimidobutyryloxy-succinimide ester, N-hydroxy sulfosuccinimidyl-4-azidobenzoate, N-succinimidyl [4-azidophenyl]-1,3'-dithiopropionate, N-succinimidyl [4-iodoacetyl]aminobenzoate, glutaraldehyde, and succinimidyl-4-[N-maleimidomethyl]cyclohexane-1-carboxylate, 3-(2-pyridyldithio)propionic acid N-hydroxysuccinimide ester (SPDP), 4-(N-maleimidomethyl)-cyclohexane-1-carboxylic acid N-hydroxysuccinimide ester (SMCC), and the like.

However method of attachment are not limited to such covalent means, and the two parts of the probe may be joined in any desired or convenient way, including through the intermediacy of groups or elements which may provide for non-covalent attachment of the nucleic acid moiety to the analyte-binding moiety. Thus, the nucleic acid moiety may be attached to the analyte-binding moiety via an intermediate group or element. By way of representative example, the nucleic acid moiety and analyte binding moiety of the first proximity probe may be linked indirectly in a non-covalent way, by means of an intermediate nucleic acid sequence; a "linker" oligonucleotide may be attached to the analyte-binding moiety (for example by any means as discussed above) and the nucleic acid moiety may be hybridised to this. Thus, if it is desired that the nucleic acid moiety be attached to the analyte-binding moiety by its 5' end, and that it accordingly have a "free" 3' end (e.g. free for extension, although, as discussed below, this may in some embodiments be "blocked"), the linker oligonucleotide may be attached to the analyte-binding moiety (e.g. covalently) by its 3' end, and the nucleic acid moiety may hybridise to this, leaving an overhanging 3' end (i.e. a free or optionally blocked, but nonetheless single stranded 3' end). Conversely, the linker oligonucleotide may be attached to the analyte-binding moiety at its 5' end and the nucleic acid moiety may hybridise to this leaving an overhanging 5' end.

The first proximity probe of the invention may be prepared using any convenient method. In representative embodiments, a nucleic acid moiety may be conjugated to the analyte-binding domain, either directly or through a linking group. The components can be covalently bonded to one another through functional groups, as is known in the art, where such functional groups may be present on the components or introduced onto the components using one or more steps, e.g. oxidation reactions, reduction reactions, cleavage reactions and the like. Functional groups that may be used in covalently bonding the components together to produce the proximity probe include: hydroxy, sulfhydryl, amino, and the like. The particular portion of the different components that are modified to provide for covalent linkage may be chosen so as not to substantially adversely interfere with that component's desired binding affinity for the target analyte. Where necessary and/or desired, certain moieties on the components may be protected using blocking groups, as is known in the art, see e.g. Green & Wuts, Protective Groups in Organic Synthesis (John Wiley & Sons) (1991). Methods for producing nucleic acid/antibody conjugates are well known to those of skill in the art.

In other embodiments, the first proximity probe may be produced using *in vitro* protocols that yield nucleic acid-protein conjugates, i.e. molecules having nucleic acids, e.g. coding sequences, covalently bonded to a protein, i.e. where the analyte-binding moiety is produced *in vitro* from vectors which encode the proximity probe. Examples of such *in vitro* protocols of interest include: RepA based protocols (see e.g., Fitzgerald, DDT (2000) 5:253-258_and WO 98/37186), ribosome display based protocols (see e.g., Hanes et al., Proc. Natl Acad. Sci. USA (1997) 94:4937-42; Roberts, Curr Opin Chem Biol 1999 Jun; 3(3): 268-73; Schaffitzel et al., J Immunol Methods 1999 Dec 10; 231 (1-2): 119-35; and WO 98/54312), etc.

As described in step (a)(i) above, the nucleic acid moiety of the first proximity probe hybridises to a circular or circularisable (linear) oligonucleotide. Such hybridisation may occur before the step of contacting the sample with the at least one set of proximity probes (i.e. the circular or circularisable oligonucleotide may be pre-hybridised to the nucleic acid moiety of the first proximity probe); or may occur at the same time as the contacting step (i.e. the sample may be contacted with the circular or circularisable oligonucleotide at the same time as being contacted with the at least one set of proximity probes); or may occur subsequently (i.e. the sample may be contacted with the circular or circularisable oligonucleotide after the sample has been contacted with the at least one set of proximity probes). By "circular" in this context it is meant that the nucleic acid molecule is in a circular conformation, with no free ends. Reference herein to a "circularisable " oligonucleotide means a nucleic acid in linear (non-circular) conformation which has free 5' and 3' ends and is thereby amenable to intramolecular or intermolecular ligation resulting in the adoption of a circular conformation (i.e. the linear nucleic acid may become circularised). It will be understood that for circularisation (ligation) to occur, the circularisable oligonucleotide has a 5' phosphate group.

In order to undergo a productive hybridisation, as defined above, with the nucleic acid moiety of the first proximity probe, the circular or circularisable oligonucleotide must contain a portion complementary to a portion of said nucleic acid moiety, wherein said portion must be of a length sufficient to allow a productive hybridisation. Suitable lengths for such a portion are discussed above, in the context of suitable lengths of the corresponding (e.g.terminal) portion of the nucleic acid moiety. In the case of a circular oligonucleotide, the portion complementary to the nucleic acid moiety of the first proximity probe may be located at any point in the circular oligonucleotide. If the oligonucleotide is linear (non-circularised; "circularisable") the complementary portion must be "divided" between the 5' and 3' ends of the oligonucleotide such that there is a sufficient length of complementarity at each of these ends to mediate a productive hybridisation of both ends to the nucleic acid moiety. "Productive hybridisation" in this particular sense therefore imparts the additional requirement (further to the definition given above relating to the facilitation of RCA) that both ends of the oligonucleotide must be able to hybridise to the corresponding portion of the nucleic acid moiety and thereby become ligated, directly or indirectly, to each other resulting in circularisation of the oligonucleotide. "Direct" in this context means that the ends of the oligonucleotide hybridise immediately adjacently on the nucleic acid moiety to form a substrate for a ligase enzyme, resulting in their ligation to each other (intramolecular ligation). Alternatively, "indirect" means that the ends of the oligonucleotide hybridise non-adjacently to the nucleic acid moiety, i.e. separated by one or more intervening nucleotides. In such an embodiment said ends are not ligated directly to each other, but circularisation of the oligonucleotide instead occurs either via the intermediacy of one or more intervening (so-called "gap" or "gap-filling" (oligo)nucleotides) or by the extension of the 3' end of the oligonucleotide to "fill" the "gap" corresponding to said intervening nucleotides (intermolecular ligation). Thus, in the former case, the gap of one or more nucleotides between the hybridised ends of the circularisable oligonucleotide may be "filled" by one or more "gap" (oligo)nucleotide(s) which are complementary to the intervening part of the portion of the nucleic acid moiety. Circularisation of the circularisable oligonucleotide thereby involves ligation of the ends of the oligonucleotide to at least one gap (oligo)nucleotide, such that the gap (oligo)nucleotide becomes incorporated into the resulting circularised molecule. Hence, in such an embodiment the template for the RCA of step (c) above contains the circularisable oligonucleotide and said gap (oligo)nucleotide. In such an embodiment, the intervening part of the portion of the nucleic acid moiety may be of any length sufficient to allow a productive hybridisation with the gap oligonucleotide, wherein by productive hybridisation in this context is meant a hybridisation capable of templating the indirect ligation (i.e. via the gap oligonucleotide) of the ends of the circularisable oligonucleotide. The intervening part of the portion of the nucleic acid moiety may be of any sequence, providing the sequence does not cause hybridisation with the circularisable oligonucleotide. The gap oligonucleotide may contain sequences useful for amplification or detection or sequencing, etc, of the eventual RCA product. Additionally or alternatively, the gap oligonucleotide may contain one or more tag or barcode sequences (discussed below). It will be seen that in a related embodiment more than one gap oligonucleotide might be used, which gap oligonucleotides hybridise to the intervening part of the portion of the nucleic acid moiety in such a way that they, and the ends of the circularisable oligonucleotide, are ligated together end-to-end during the ligation step.

In the latter case, the gap between the ends of the circularisable oligonucleotide hybridised to the portion of the nucleic acid moiety may be filled by polymerase-mediated extension of the 3' end of the circularisable oligonucleotide. Suitable polymerases are known in the art. Once said 3' end has been extended as far as the 5' end of the linear nucleic acid molecule, the ends may be joined in a ligation reaction.

Thus, in an embodiment wherein the oligonucleotide is linear, the nucleic acid moiety acts to template the intra- or intermolecular ligation of the circularisable oligonucleotide, causing it to become circularised. Since, as will be readily appreciated by those of skill in the art, RCA as specified in step (c) above requires a circular ("circularised") template, in any embodiments in which the template oligonucleotide of step (a)(i) above is linear (non-circularised; "circularisable") said molecule must be circularised by said intra- or intermolecular ligation during step (b) of the method.

The circular or circularisable oligonucleotide may be of any suitable length to act as an RCA template. Thus for example it may be at least 20 nucleotides long, e.g. at least 25, 30, 32, 35, 36, 37, 38, 39 or 30 nucleotides long. In addition to the portion, or (end) portions in the case of a circularisable linear oligonucleotide, complementary to the nucleic acid moiety of the first proximity probe, the oligonucleotide may contain features or sequences or portions useful in the detection or further amplification of the RCA product. Such sequences may include binding sites for hybridisation probes and/or amplification or sequencing primers. The circular or circularisable linear oligonucleotide may additionally or alternatively contain arbitrary "tag" or "barcode" sequences which may be used diagnostically to identify the analyte to which a given RCA product relates, in the context of a multiplex assay. Such a sequence is simply a stretch of nucleotides comprising a sequence designed to be present only in the circular or circularisable linear oligonucleotide which is "specific for" (i.e. capable of hybridising only to the nucleic acid moiety of the probe pair which is specific for) a particular analyte. Hence, where the presence or amount of a plurality of different analytes is simultaneously assayed, respective probe sets for the various analytes are associated with a particular circular or circularisable linear oligonucleotide by virtue of the required portion of complementarity between the latter and the nucleic acid moiety of the first proximity probe of each pair. Thus, for a given first proximity probe for a particular analyte in a multiplex assay, only one (species of) circular or circularisable linear oligonucleotide will have the requisite complementarity. The presence, in the circular or circularisable linear oligonucleotides for the respective analyte-specific probe pairs, of different arbitrary tag or barcode sequences allows the detection of a particular RCA product (for example by hybridisation of labelled probes to said arbitrary sequences) to be understood as being indicative of the presence or amount of a particular one of several assayed analytes.

The enzyme moiety of the second proximity probe may be any enzyme which is capable, directly or indirectly, of enabling (or alternaively put of facilitating or mediating) RCA of the circular or circularisable oligonucleotide. As discussed above, in a preferred embodiment the RCA uses the nucleic acid moiety of the first proximity probe as primer, but the RCA primer may alternatively be hybridised to the nucleic acid moiety. The enzyme moiety may thus be an enzyme that acts on the nucleic acid moiety of the first proximity probe and/or on the oligonucleotide which hybridises to it. It may accordingly alternatively be more broadly defined as an enzyme moiety capable of acting on nucleic acid. The term "acts on" is used broadly herein to include any interaction or association of the enzyme with the nucleic acid (e.g. the nucleic acid moiety or the oligonucleotide). The enzyme moiety may thus use the nucleic acid in any way, for example as substrate, or template, or primer etc.

"Direct" enablement of RCA may be understood to mean that the enzyme moiety is involved in the process of RCA, i.e. is a polymerase suitable for performing RCA. Such polymerases are known in the art and include phi29 polymerase, Klenow fragment or BST. The person skilled in the art may readily determine other suitable polymerases which might be used. In such an embodiment, the first and second probes bind to the analyte, if present in the sample, and thereby come into close proximity. If the oligonucleotide of step (a)(i) above which serves as (or to provide or form) the RCA template is provided as a circularisable linear, rather than a circular, molecule, an additional step involving the provision of a ligase enzyme, and optionally also one or more gap oligonucleotides or a polymerase and nucleotides for filling of any gap between the ends of the circularisable oligonucleotide when hybridised to the nucleic acid moiety (discussed above), will be necessary in order to render the oligonucleotide circularised so that it can template RCA. (It may also be necessary to provide in this step other reagents and/or conditions necessary for a ligation reaction, as in known in the art e.g. ATP). After the circular or circularisable oligonucleotide has hybridised to the nucleic acid moiety of the first proximity probe (and, if necessary, has become circularised in an additional ligation step), the polymerase enzyme moiety of the second proximity probe may act to catalyse RCA using the circular/circularised oligonucleotide as template. The RCA reaction is directly or indirectly primed by the nucleic acid moiety. For example, the polymerase may extend the 3' end of the nucleic acid moiety using the circular/circularised oligonucleotide as template. Alternatively, a primer may be hybridised to a 3' attached nucleic acid moiety, leaving a free 3' end to prime the RCA. As a result of RCA, concatemeric amplification products containing numerous tandem repeats of the oligonucleotide are produced and may be detected as indicative of the presence or amount of analyte in the sample. In the absence of analyte in the sample, the polymerase enzyme does not come into sufficiently close proximity to the first proximity probe-circularlcircularised oligonucleotide complex to enable appreciable levels of RCA. The fact that the polymerase is in this embodiment brought into proximity with the nucleic acid moiety of the first proximity probe, rather than being added free in solution, offers the advantage that less enzyme is required to be used relative to the above-discussed embodiment of WO 99/49079 wherein both probes have nucleic acid moieties. The "tethering" of the enzyme may also result in a more rapid assay.

Accordingly, in a particular embodiment of the invention there is provided a method for localised *in situ* detection of an analyte in a sample, comprising:
(a) contacting said sample with at least one set of at least first and second proximity probes, wherein said probes each comprise an analyte-binding moiety and can simultaneously bind to the analyte, and wherein
   (i) said first proximity probe comprises a nucleic acid moiety attached at one end to the analyte-binding moiety, wherein a circular or circularisable oligonucleotide is hybridised to said nucleic acid moiety before, during or after said contacting step; and
   (ii) said second proximity probe comprises a polymerase enzyme moiety, attached to the analyte-binding moiety, capable of performing rolling circle amplification (RCA) of the circular or, when it is circularised, of the circularisable oligonucleotide hybridised to the nucleic acid moiety of the first proximity probe wherein said RCA is primed by said nucleic acid moiety of said first proximity probe;
(b) circularising said oligonucleotide, if necessary, to produce a circularised template for RCA;
(c) subjecting said circular or circularised template to RCA using said polymerase moiety of the second proximity probe and wherein the nucleic acid moiety of the first proximity probe directly or indirectly mediates the priming of the RCA reaction; and
(d) detecting a product of said RCA.

Thus in this embodiment of the invention, the RCA is carried out by the polymerase which is provided on the second proximity probe, and accordingly in this embodiment the RCA step, step (c), does not require, or does not utilise, a free DNA polymerase, or a polymerase added to the reaction.

In the method of this embodiment the nucleic acid moiety of the first proximity probe directly or indirectly mediates priming of the RCA reaction, for example either by acting as a primer, or by providing a binding site for a primer. In other words the polymerase in the RCA reaction uses as a primer the nucleic acid moiety of the first proximity probe, or a primer oligonucleotide hybridised thereto. Accordingly, in one version of this embodiment the nucleic acid moiety may be attached at its 5' end to the analyte-binding moiety of the first probe and may act as the primer for the RCA of the circular or circularisable probe. In such a case, the polymerase of the second probe (in step a(ii)) may be capable of performing said RCA of the circular or circularized oligonucleotide using said nucleic acid moiety of said first proximity probe as a primer (alternatively put, by extending said nucleic acid moiety of said first proximity probe by which said RCA is primed); in step (c) the RCA is carried out using the nucleic acid moiety of the first probe as primer. Thus in this version of the method, it will be understood that in step (c) the RCA is primed by said nucleic acid moiety of said first proximity probe.

In an alternative version of this embodiment of the method, the nucleic acid moiety may be attached by its 3' end to the nucleic acid binding moiety of the first proximity probe, and a primer oligonucleotide may be hybridised to the nucleic acid moiety (e.g. at or around (near) its free 5' end, e.g. within 3, 4, 5, 6, 8, 10 or 12 nucleotides of the 5' end, or at least overlapping the 5' end) so as to provide a free 3' end which may act as a primer for the RCA step. In such a case, the polymerase of the second probe is capable of performing said RCA of the circular or circularised oligonucleotide using a primer oligonucleotide hybridised to the nucleic acid moiety as primer; in step (c) the RCA is performed using as primer a primer oligonucleotide hybridised to the nucleic acid moiety and having a free 3' end. As noted above, the primer oligonucleotide may be hybridised to the nucleic acid moiety before, during or after the contacting step.

"Indirect" enablement (or facilitation etc.) of RCA encompasses any other mechanism by which an enzyme moiety attached to the analyte-binding moiety of the second proximity probe can act to enable RCA of the circular or circularisable oligonucleotide using the (3' end of) the nucleic acid moiety of the first proximity probe as primer or using a primer oligonucleotide hybridised to the nucleic acid moiety as primer), wherein due to features of the reagents of step (a)(i) above (i.e. the first proximity probe and circular or circularisable oligonucleotide) such RCA could not occur in the absence of the particular enzyme moiety.

In one such embodiment, the enzyme moiety is a ligase. In such an embodiment, the oligonucleotide of step (a)(i) above which serves as the RCA template is provided as a circularisable linear, rather than as a circular (i.e. an already or "pre-"circularised) molecule. As discussed above, the circularisable linear oligonucleotide may be provided pre-hybridised to the nucleic acid moiety of the first proximity probe, or may be contacted with the sample at the same time as, or later than, the first proximity probe is contacted with the sample. In the presence in the sample of analyte, binding of the respective probes to the same analyte molecule brings the ligase-bearing second proximity probe close to the nucleic acid moiety of the first proximity probe to which is hybridised said circularisable oligonucleotide. The ligase enzyme moiety acts to ligate, directly or indirectly (as discussed above), the ends of the circularisable oligonucleotide which are hybridised immediately adjacent, or with a gap of one or more intervening nucleotides, on the nucleic acid moiety oligonucleotide which can serve as an RCA template. RCA is effected by the subsequent provision of a polymerase enzyme capable of mediating RCA, as are known in the art and discussed above, and a detectable RCA product is generated. In the absence of analyte in the sample, the ligase enzyme does not come into close enough proximity to the first proximity probe to circularise (ligate) the circularisable oligonucleotide hybridised thereto, and therefore no circularised RCA template is made to serve as a substrate for the polymerase. RCA does not occur at appreciable levels in the absence of analyte. The fact that the ligase is in this embodiment brought into proximity with the nucleic acid moiety of the first proximity probe, rather than being added free in solution, offers the advantage that less enzyme is required to be used relative to the above-discussed embodiment of WO 99/49079 wherein both probes have nucleic acid moieties. The "tethering" of the enzyme may also result in a more rapid assay.

Hence, a further particular embodiment of the invention provides a method for localised *in situ* detection of an analyte in a sample, said method comprising:
(a) contacting said sample with at least one set of at least first and second proximity probes, wherein said probes each comprise an analyte-binding moiety and can simultaneously bind to the analyte, and wherein
   (i) said first proximity probe comprises a nucleic acid moiety attached at one end to the analyte-binding moiety, wherein a circularisable linear oligonucleotide is hybridised to said nucleic acid moiety before, during or after said contacting step; and
   (ii) said second proximity probe comprises a ligase enzyme moiety, attached to the analyte-binding moiety, capable of circularising said oligonucleotide;
(b) circularising said oligonucleotide using said ligase enzyme moiety to produce a circularised template for RCA;
(c) subjecting said circularised template to RCA wherein said RCA is primed by said nucleic acid moiety of said first proximity probe; and
(d) detecting a product of said RCA.

In this embodiment, by enabling the formation of a circularised template the ligase enzyme acts to enable (or to facilitate) rolling circle amplification (RCA) of the circularisable linear nucleic acid molecule hybridised to the nucleic acid moiety of the first proximity probe.

Analogously to the first ("polymerase") embodiment described above, the nucleic acid moiety of the first proximity probe directly or indirectly mediates priming of the RCA reaction. Thus, it may either be 5'-attached to the analyte-binding moiety of the probe and may act as primer for the RCA, or it may be 3' -attached and may provide a binding site for a primer oligonucleotide which hybridises to the nucleic acid moiety (e.g. at or around its free 5' end) and has a free 3' end which may act (or be used) as primer for the RCA. Thus, in step (c) of this embodiment, the RCA may be primed by said nucleic acid moiety of said first proximity probe (i.e. when it is 5'-attached to the analyte-binding moiety of the first probe) or, when the nucleic acid moiety is 3'-attached, the RCA may be performed using as primer a primer oligonucleotide hybridised to the nucleic acid moiety and having a free 3' end. As noted above, the primer oligonucleotide may be hybridised to the nucleic acid moiety before, during or after the contacting step.

In another embodiment of the method wherein the enzyme moiety of the second proximity probe indirectly enables RCA, the enzyme moiety is a nucleic acid cleaving enzyme. Such an enzyme may be a restriction enzyme, or other endonuclease or an exonuclease.

As in the case of the "direct" RCA-enabling embodiment described above, in such an embodiment the oligonucleotide of step (a)(i) above, which will serve as (or form or provide) the RCA template, may be provided in circular or circularisable linear form, and may be pre-hybridised to the nucleic acid moiety of the first proximity probe or may be contacted with the sample at the same time as, or later than, the first proximity probe is contacted with the sample. If said oligonucleotide is provided in circularisable linear form an additional step involving the provision of a ligase enzyme, and optionally also one or more gap oligonucleotides or a polymerase and nucleotides for filling of any gap between the ends of the oligonucleotide when hybridised to the nucleic acid moiety (discussed above), will be necessary in order to render the molecule circularised so that it can template RCA. In such an embodiment involving a nucleic acid cleaving enzyme moiety, the nucleic acid moiety of the first proximity probe is attached at its 5' end to the analyte-binding moiety (i.e. has a "free" 3' end) and is modified at the 3' terminal portion (i.e. the end of the nucleic acid moiety other than that by which it is connected to the analyte-binding domain) such that extension from the 3' end of the nucleic acid moiety, such as by a polymerase, is "blocked". The presence of the blocking group therefore prevents the nucleic acid moiety from serving as RCA primer. Suitable blocking groups, or reagents to perform the blocking modification, are well known to those of skill in the art and are described further below. In the presence in the sample of analyte the nucleic acid cleaving enzyme moiety of the second probe is brought into proximity to the blocked nucleic acid moiety of the first probe, to which is hybridised the circularised or circularisable oligonucleotide, as a result of the same analyte being bound by both probes. The nucleic acid cleaving enzyme moiety acts to cleave off the blocking groups, rendering the 3' terminal portion of the nucleic acid moiety capable of priming RCA, using as template the oligonucleotide (which, if necessary, has become circularised in an additional ligation step), upon the provision of a suitable polymerase (such polymerases being known in the art, as discussed above). A detectable RCA product is thereby generated. In the absence of analyte in the sample, the nucleic acid cleaving enzyme does not come into close enough proximity to the first proximity probe to remove the blocking groups. Therefore, no primer is present to effect RCA, which does not occur at appreciable levels in the absence of analyte.

Thus, in a further particular embodiment the invention provides a method for localised *in situ* detection of an analyte in a sample, comprising:
(a) contacting said sample with at least one set of at least first and second proximity probes, wherein said probes each comprise an analyte-binding moiety and can simultaneously bind to the analyte, and wherein
   (i) said first proximity probe comprises a nucleic acid moiety attached at its 5' end to the analyte-binding moiety, wherein said nucleic acid moiety contains a modification at or near its 3' end which blocks polymerase-mediated extension, and wherein a circularised or circularisable linear oligonucleotide is hybridised to said nucleic acid moiety before, during or after said contacting step; and
   (ii) said second proximity probe comprises a nucleic acid cleaving enzyme moiety, attached to the analyte-binding moiety, capable of cleaving off said modification; thereby allowing polymerase-mediated extension of said 3' end;
(b) circularising said oligonucleotide, if necessary, to produce a circularised template for RCA;
(c) subjecting said circular or circularised template to RCA, wherein said RCA is primed by said nucleic acid moiety of said first proximity probe; and
(d) detecting a product of said RCA.

In this embodiment the nucleic acid cleaving enzyme enables rolling circle amplification (RCA) of the circular or, when it is circularised, of the circularisable oligonucleotide hybridised to the nucleic acid moiety of the first proximity probe by providing a primer for the RCA. More particularly, by removing a blocking modification from the 3' end (i.e. unblocking, releasing or "freeing" the 3' end) of the nucleic acid moiety, it is capable of being extended by a polymerase and hence of acting (or serving) as a primer for the RCA of step (c).

Upon binding of said probes to the analyte in step (a), the enzyme is able (or allowed) to cleave said nucleic acid moiety to remove the modification (i.e. the blocking group). Thus, it will be seen that in this embodiment the method includes the step of allowing or permitting said enzyme to cleave said nucleic acid moiety of said first proximity probe to remove said modification (or more simply put, the step of cleaving off said modification, or using the enzyme to cleave off the modification).

In this regard, it will be appreciated that the nucleic acid moiety may be provided with one or more modifications (e.g. blocking groups, or phosphodiester bond modifications) and hence the term "a modification" comprises one or more modifications.

As discussed above, he "5'-attachment" of the nucleic acid moiety to the analyte-binding moiety of the first proximity probe include direct attachment or indirect attachment, e.g. via hybridisation to a linker oligonucleotide which is itself 3'-attached (e.g. covalently) to the analyte binding moiety.

Examples of blocking groups, or blocking modifications, include but are not limited to, terminal 2'O Me-RNA , Locked Nucleic Acids, Peptide Nucleic Acids, teminator groups such as dideoxy nucleotides (ddNTPs) or other modified nucleotides or using an inverse 3'-end to produce a 5'-end instead using reagents such as Inverse dT phosphoamidities during synthesis. To ensure a good block it may in certain instances be advantageous to use several modified residues in the 3' end, such as 2,3,4,5 or 6 LNA, PNA or 2'O Me RNA residues in a row for example. In the literature various modifications of the 3'ends of oligonucleotides have been described and any modification that prevents a polymerase from recognizing the end as an 3'end is functional in this context. The exact function of a particular modification may differ depending on the polymerase used, mainly due to the exo/endonuclease activity of some polymerases that can cleave off the modification. Therefore it is often advantageous to use several (more than one) modified nucleotides at the 3' end if the polymerase has such an activity, as is the case for phi29 polymerase.

In order for the cleavage by the nucleic acid cleaving enzyme to take place the nucleic acid moiety may be provided with a cleavage site for the enzyme. For example, where the nucleic acid cleaving enzyme is a restriction endonuclease, this may be a restriction cleavage site. It will be understood therefore that in this embodiment the nucleic acid moiety may be partially double-stranded to enable the provision of a cleavage site (in the double-stranded portion). This may be located appropriately to allow the blocking modification to be removed (cleaved off).

Alternatively, the nucleic acid cleaving enzyme may be a uracil DNA glycosylase (UDG), and the cleavage site may be one or more uracil (U) residues, e.g. a stretch of 1 to 6 U residues.

Other possibilities include the use of a nicking endonuclease, which is described in the literature, or apurinic/apyrimidinic endonuclease to cleave apurinic/apyrimidic sites.

Reversible terminators are well-described in the literature and have been used in sequencing/minisequencing applications. Any such reversible terminator group may be used. Removal of a terminator can be performed using normal chemical reactions such as thiol-cleavage, oxidation and reduction but it will be understood that in the method of the invention the terminator will be removed by the action of the nucleic acid cleaving enzyme. Reversible terminators are described, *inter alia*, in :Guo et al., Acc Chem Res. 2010 Feb 3. [Epub ahead of print]PMI D: 20121268; Bowers et a/., Nat Methods. 2009 Aug;6(8):593-5. Epub 2009 Jul 20.PMID: 19620973; Knapp et a/., Nucleic Acids Symp Ser (Oxf). 2008;(52):345-6.PMID: 18776395; Guo et al., Proc Natl Acad Sci U S A. 2008 Jul 8;105(27):9145-50. Epub 2008 Jun 30.PMID: 18591653; Turcatti et al., Nucleic Acids Res. 2008 Mar;36(4):e25. Epub 2008 Feb 7.PMID: 18263613; Wu et al., Proc Natl Acad Sci U S A. 2007 Oct 16;104(42):16462-7. Epub 2007 Oct 8.PMID: 17923668; Földesi et al., Nucleosides Nucleotides Nucleic Acids. 2007;26(3):271-5.PMID: 17454736; Meng et a/., J Org Chem. 2006 Apr 14;71 (8):3248-52.PMID: 16599623; Ruparel et al., Proc Natl Acad Sci U S A. 2005 Apr 26;102(17):5932-7. Epub 2005 Apr 13.PMID: 15829589

The above-discussed ligase and nucleic acid cleaving enzyme embodiments of the indirect-RCA-facilitation embodiment of the method of the invention are merely non-limiting examples of enzyme moieties which may be used in the method of the invention as defined generally above. In light of the general principle disclosed herein of the use of a proximity probe pair for analyte detection wherein one probe of the pair carries an enzyme moiety, the skilled person may readily envisage alternative embodiments using different enzyme moieties on the second proximity probe.

As is apparent from the above-described embodiments the generation, or not, of a detectable, quantifiable RCA product can be made analyte-dependent through the use of proximity probes wherein one such probe comprises an enzyme moiety capable of directly, or indirectly, facilitating RCA. In particular, such an enzyme is required in order for the RCA to take place. As can be seen from the examples above, this can be by providing (or enabling the provision of e.g. generating) reagents or substrates for the RCA, including as illustrated above, the RCA template or the primer for the RCA.

The two moieties of the second proximity probe are joined together by any convenient or available means, such as are widely known and reported in the art. The coupling of enzymes to proteins is a well-established process. For example, covalent linking of enzymes such as horseradish peroxidase to antibodies has been performed for many years.

In step (c) of the methods above, RCA is performed using the circular or circularisable oligonucleotide of step (a)(i) above as template. As discussed above, since RCA requires a circular (or"circularised") template, in embodiments in which the nucleic acid molecule of step (a)(i) above is provided in linear (non-circularised) form, said molecule is circularised prior to step (c) of the method (see step (b) of the methods above). Hence, reference to subjecting the circularisable oligonucleotide to RCA means performing RCA using as template a circularised oligonucleotide deriving from said circularisable oligonucleotide, which circularised oligonucleotide in some embodiments may contain "intervening" sequence complementary to a part of of the nucleic acid moiety of the first proximity probe which is located between parts of said moiety which are complementary (and hybridise) to the ends of the circularisable oligonucleotide. As discussed above, such intervening sequence may be incorporated into the circularisable oligonucleotide through ligation to one or more gap oligonucleotides or by extension of the 3' end of the oligonucleotide using the above-mentioned part of the nucleic acid moiety as template.

Step (b) of the methods of the invention is thus performed if necessary. It will be necessary in embodiments in which the oligonucleotide which is hybridised to the nucleic acid moiety of the first proximity probe is provided in non-circular form, i.e. when it is a linear "circularisable" oligonucleotide. In embodiments wherein the enzyme moiety is a ligase, step (b) is performed by the ligase of the second proximity probe. As discussed above, ligation may require additional reagents and/or conditions etc to be provided, and this may be applicable also to ligation performed by a ligase provide by the second proximity probe. In other embodiments, where the enzyme moiety of the second proximity probe is not a ligase, this step will be performed by the addition of a ligase enzyme (i.e. a separate, discrete, step of circularising the oligonucleotide may be performed (a separate, discrete, ligation step). Thus, there may be a step of adding ligase e.g. to the reaction mixture.

In embodiments of the method of the invention in which RCA is "directly" mediated by a polymerase enzyme moiety on the second proximity probe, it is not appropriate to add "free" (i.e. not comprised as part of the second proximity probe) polymerase to effect RCA, as indicated in step (b) above. In "indirect" embodiments (i.e. when the enzyme moiety is not a polymerase enzyme), step (b) involves providing all reagents (e.g. nucleotides, cofactors) and conditions (e.g. appropriate temperature) for RCA, in addition to a suitable polymerase. The required reagents and conditions for RCA are well known in the art. In "direct" embodiments, a polymerase capable of performing RCA is omitted from this step, though all other reagents and conditions must be provided to allow the polymerase of the second proximity probe to effect RCA.

As described in step (d) above, following the RCA of step (c) and the generation of a concatemeric RCA product, said product is detected. As explained above, the RCA product is localised to the analyte (since it is attached to (in certain embodiments, the product is "attached" by virtue of being continuous with) the nucleic acid moiety of the first proximity probe, which is bound to the analyte). Thus localised detection of the analyte is rendered possible. This in turn enables the analyte to be detected *in situ.*

Detection may be by any suitable means for the localised (i.e. indicative of spatial position within the sample) and qualitative (presence or absence) or quantitative (presence and amount, or absence) detection of said product. Localisation, (i.e. localised detection) may be defined at a sub-cellular (e.g. wherein the cell is the analyte localised) or cellular level. Thus, different cells may be analysed or distinguished, e.g. whether the signal originates from a particular cell (for example cell A or cell B). As RCA produces a continuous product comprising tandem repeats of the circular template (the circular or circularised oligonucleotide of step (a)(i)), the number of repeats being a function of the length of the template and the duration of the isothermal amplification reaction, it can provide absolute or relative quantitative information on the amount of analyte present in the sample. Hence, providing the proximity probes are present in excess, the number of RCA products generated for a given analyte species should be proportional to the number of analyte molecules of that species present in a sample. Providing a detection method is used which reflects the number of RCA products present for a given analyte, the amount of analyte in the sample may be determined. Such quantitative detection may be relative, by comparison between the RCA products of different analytes in a multiplex assay, or absolute, by inclusion in the RCA step of a known amount of control template of known size (preferably the same size as the "analyte-specific" template corresponding to the circular or circularised oligonucletide of step (a)(i)).

As discussed above, the RCA product is attached to (e.g. is continuous with) the nucleic acid moiety of the first proximity probe, which either primes the RCA or is hybridised to the RCA primer, and as a consequence is anchored to the analyte molecule via the analyte-binding domain of the probe. The physical attachment of the detectable RCA product for a particular analyte to that analyte crucially facilitates retention of the detectable product to the spatial position of the analyte within the sample, causing the detection to be localised.

The localised detection may be viewed as comprising two steps, firstly the development of a detectable signal and secondly the read-out of the signal. With respect to the first step the following detection methods could be contemplated: The signal may include, but is not limited to a fluorescent, chromogenic, radioactive, luminescent, magnetic, electron density or particle-based signal. Thus, a label directly or indirectly providing such a signal may be used. The signal could be obtained either by incorporating a labelled nucleotide during amplification to yield an labelled RCA product, using a complementary labelled oligonucleotide that is capable of hybridization to the RCA product, or to, in a sequence non-specific manner, label the produced nucleic acid. The label could be direct, (e.g. but not limited to: a fluorophore, chromogen, radioactive isotope, luminescent molecule, magnetic particle or Au-particle), or indirect (e.g. but not limited to an enzyme or branching oligonucleotide). The enzyme may produce the signal in a subsequent or simultaneous enzymatic step. Several methods are well described in the literature and are known to be used to render signals that are detectable by various means (which may be used in the second step) e.g. microscopy (bright-field, fluorescent, electron, scanning probe)), flow cytometry (fluorescent, particle, magnetic) or a scanning device.

In a particular embodiment, detection is by means of labelled oligonucleotide probes which have complementarity, and thereby hybridise, to the RCA product. Such labelling may be by any means known in the art, such as fluorescent labelling including ratiolabelling, radiolabelling, labelling with a chromogenic or luminescent substrate etc. Fluorescently-labelled probes are preferred. The signal produced by the labels may be detected by any suitable means, such as visually, including microscopically. As the RCA products are comprised of repeated "monomers" corresponding to the circular or circularised oligonucleotide of step (a)(i) (optionally with additional incorporated nucleotides or gap oligonucleotides, as discussed above; the "circularised template"), the sequences to which the oligonucleotide probes hybridise will be "repeated", i.e. assuming the RCA reaction proceeds beyond a single replication of the template, multiple sites for hybridisation of the oligonucleotide probes will exist within each RCA product. In this way, the signal intensity from the label on the oligonucleotide probes may be increased by prolonging the RCA reaction to produce a long RCA product containing many hybridisation sites. Signal intensity and localisation is further increased due to spontaneous coiling of the RCA product. The resulting coils, containing multiple hybridised oligonucleotide probes, give a condensed signal which is readily discernible by, for example, microscopic visualisation against a background of non-hybridised oligonucleotide probes. Hence, it may be possible qualitatively or quantitatively to detect the analytes(s) in a sample without performing a washing step to remove unhybridised oligonucleotide probes.

Multiplexed detection may be facilitated by using differently-labelled oligonucleotide probes for different analytes, wherein the respective oligonucleotide probes are designed to have complementarity to "unique" sequences present only in the RCA products (corresponding to sequences present only in the circular or circularised templates) for the respective analytes. Such sequences may be barcode or tag sequences, as discussed above.

The invention provides a kit for use in the method of the invention. The kit will comprise at least one (species of) first and second proximity probe, as defined above, specific for a particular analyte. Thus, the first proximity probe will comprise an analyte-binding moiety and a nucleic acid moiety, whilst the second binding moiety will comprise an analyte-binding moiety and an enzyme moiety, such as a polymerase or ligase or cleaving enzyme moiety. The analyte-binding moiety may be a binding partner capable of directly binding the analyte ("direct analyte-binding moiety"), such as a primary antibody, or it may be a moiety with binding specificity for such a primary binding partner, for example a secondary antibody with specificity for a primary analyte-binding antibody ("indirect analyte-binding moiety"). In one embodiment wherein the proximity probes of the kit contain indirect analyte-binding moieties, the kit further comprises analyte-binding partners for which the indirect analyte-binding moieties of the probes have .binding specificity.

Kits comprising the nucleic acid moiety of the first probe, and/or the enzyme moiety, of the second probe, optionally together with reagents for attaching the said moieties to an analyte-binding moiety are described herein. In this way, the user of the kit may conveniently be able to attach the nucleic acid and enzyme moieties to his "own" analyte-binding moieties to form "customised" proximity probes, according to choice. As noted above, such a kit may also comprise reagents for attaching the nucleic acid and/or enzyme moieties to an analyte-binding moiety.

The kit may further comprise a circular or circularisable oligonucleotide as defined above, comprising a portion having functional complementarity with the nucleic acid moiety of the first proximity probe of the kit (e.g. at the 3' terminal portion of the nucleic acid moiety). In such a kit, the circular or circularisable oligonucleotide may be provided separately from, or pre-hybridised to, the first proximity probe (or to the nucleic acid moiety, if provided separately). If the kit contains a circularisable linear oligonucleotide, the kit may optionally further comprise one or more gap oligonucleotides with complementarity to a portion of the nucleic acid moiety of the first proximity probe of the kit, (e.g. at the 3' terminal portion of the nucleic acid moiety) or may comprise reagents for filling any gap present when the ends of the linear oligonucleotide are hybridised to the nucleic acid moiety, such as a polymerase, nucleotides and necessary co-factors.

Alternatively or additionally, the kit may comprise a ligase for circularising a circularisable linear oligonucleotide (which may or may not be present in the kit) or a polymerase such as phi29 polymerase (and optionally necessary cofactors, as well and nucleotides) for effecting RCA. Reagents for detecting the RCA product may also be included in the kit. Such reagents may include a labelled oligonucleotide hybridisation probe having complementarity to a portion of a circular or circularisable linear oligonucleotide, or to a portion of a gap oligonucleotide, present in the kit.

In a particular embodiment the kit comprises at least a first and a second proximity probe wherein the analyte-binding moiety is capable of indirectly binding the analyte via an intermediary molecule which is a direct binding partner of the analyte.

The kit may be designed for use in multiplex embodiments of the method of the invention, and accordingly may comprise combinations of the components defined above for more than one analyte. If probes having direct or indirect binding specificity respectively for a plurality of analytes are present in the kit, the kit may additionally comprise components allowing multiple analytes detection in parallel to be distinguished. For example, the kit may contain circularor circularisable oligonucleotides for use with probes specific for different analytes, wherein said respective oligonucleotides have "unique" sequences for hybridisation only to the first proximity probe for a particular analyte as well as for hybridisation (in the context of the RCA product produced by RCA templated from said nucleic acid molecules) only to a particular species of oligonucleotide hybridisation probes. Such oligonucleotide hybridisation probes respectively specific for the RCA products generated in response to the presence of particular analytes may also be provided in the kit, and may respectively carry different labels allowing the detection of different analytes to be distinguished.

In a particular embodiment the kit comprises probe sets for a plurality of analytes, in addition to circular or circularisable oligonucleotides having hybridisation specificity to the nucleic acid moiety of first proximity probes of the respective analyte-specific probe sets.

In addition to the above components, the kit may further include instructions for practicing the method of the invention. These instructions may be present in the kit in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a remote site. Any convenient means may be present in the kit.

Hence, in a further embodiment of the invention is provided a kit for localised *in situ* detection of an analyte in a sample, said kit comprising:
a set of proximity probes for at least one analyte wherein said set comprises at least a first and a second proximity probe, said first and second probes both comprising an analyte-binding moiety capable of directly or indirectly binding said analyte, wherein said probes can simultaneously bind to the analyte and wherein said first probe additionally comprises a nucleic acid moiety and said second probe additionally comprises an enzyme moiety,
and a circular or circularisable oligonucleotide comprising a portion capable of hybridising to the nucleic acid moiety of said first proximity probe, wherein said circularisable oligonucleotide is capable of being circularised when hybridised to the nucleic acid moiety of said first proximity probe,
optionally together with one or more of the following components;
   (i) if the analyte-binding moieties of said first and second probes are indirect analyte-binding moieties, direct analyte-binding moieties for which said analyte-binding moieties of the first and second probes have binding specificity;
   (ii) one or more gap oligonucleotides capable of hybridising to a portion of the nucleic acid moiety of said first proximity probe;
   (iii) a labelled oligonucleotide hybridisation probe capable of hybridising to a portion of said circular or circularisable oligonucleotide, or to a portion of said one or more gap oligonucleotides;
   (iv) a ligase;
   (v) a polymerase.

The nucleic acid moiety and the enzyme moiety will be as hereinbefore described.

Although, as described and presented herein, the invention involves the use of RCA to generate a localised signal allowing localised detection of the analyte. Other amplification methods for example, Loop Mediated Isothermal Amplification (LAMP), polymerase extension, Smart Amplification Process (SMAP) or similar are described herein. What is required is that the amplification product upon its generation remains attached, whether directly or indirectly, or otherwise associated with the first proximity probe carrying the nucleic acid moiety (e.g. in close physical association). In this way the signal generated by the interaction of the proximity probes may be localised to the analyte. Thus, in such a method the enzyme moiety of the second proximity probe may be capable of, directly or indirectly, enabling amplification of a template molecule which is hybridised to the nucleic acid moiety of the first proximity probe.

The invention will be further described with reference to the following non-limiting Examples.

### Examples

### Reagent preparation and manufacturing

The methods requires a number of bio-conjugation procedures such as coupling of oligonucleotides to ligand binders (analyte-binding moieties) e.g. antibodies and coupling of enzymes to ligand binders (e.g. antibodies). Many commercially available procedures for such work exist and are described in the literature. Any such method may be used, such as sulpho-SMCC chemistry (Pierce) which linkes primary amines on antibodies with thiol on an oligonucleotide forming a thio-ester covalent bond (Söderberg et al 2006). These reagents may additionally be purified from excess oligonucleotides using standard size-exclusion chromatography. The coupling of the enzyme moieties to the antibodies can also be accomplished using standard immunotechnology procedures and commercially available reagents (Hashida, S., et a/. (1984). More useful maleimide compounds for the conjugation of Fab' to horseradish peroxidase through thiol groups in the hinge. J. Appl. Biochem. 6, 56-63*.,* Imagawa, M., et al. (1982). Characteristics and evaluation of antibody-horseradish peroxidase conjugates prepared by using a maleimide compound, glutaraldehyde, and periodate. J. Appl. Biochem. 4, 41-57*.,* O'Sullivan, M.J., et al. (1979). Comparison of two methods of preparing enzyme-antibody conjugates: application of these conjugates for enzyme immunoassay. Anal. Biochem. 100, 100-108*.).*

### Example 1

### Assay procedure using a nucleic acid linked antibody and a DNA polymerase carrying antibody

The following protocol outlines the experimental procedure of performing the disclosed technology. An interaction between two protein is to be investigated by assessment of their close proximity using a localized readout. In this given example the interaction between the Myc protein and the Max protein is analyzed. Cultured cells grown on microscope slides are fixed using standard protocols (PFA, Actone, Zink, or other) followed by application of a blocking reagent usually containing BSA or 5-10% non-immune serum. Histological tissue samples may also be analyzed in the same procedure. A pair of target specific antibodies one for myc and the other for max are then applied to the sample. The anti-myc antibody has prior to application been conjugated with a nucleic acid component (free 3' end) while the anti-max antibody has been conjugated with a DNA polymerase enzyme, preferably phi-29 polymerase. After target binding, excess reagents are washed off and a DNA circle and dNTPs are applied to the reaction. This circular piece of DNA is complementary to the nucleic acid linked to the anti-myc antibody. In the situation where an anti-max antibody with the DNA polymerase is situated very close to the anti-may antibody, a rolling circle amplification reaction of the added circular DNA is triggered, primed from the free 3' end of the nucleic acid linked to the anti-myc antibody. After the localized RCA is performed another washing step is performed and the concatemeric repeat DNA is visualized by hybridization of a fluorescent oligonucleotide. The resulting product is inspected in a fluorescence microscope, revealing the sites of interacting myc and max protein in a localized way *in situ.*

### Example 2

### Interaction visualization enabled by the proximity of a nucleic acid carrying antibody and a DNA ligase-linked antibody.

In this experimental example, the two proximity reagents are assayed for proximity by the addition of a linear oligonucleotide with a 5' phosphate capable of hybridizing to the nucleic acid of the anti-myc antibody. The hybridization results in a nicked circular structure resembling a padlock probe reaction (Nilsson et al Science 1994). If the anti-max antibody linked to the DNA ligase is in close proximity provided by the myc/max interaction being present in the sample, the DNA ligase can seal this nick with the aid of the simultaneously added ATP. Excess reagents are washed off. The closed circular DNA formed is then replicated in a second reaction, an RCA, by the addition of a DNA polymerase and primed by the free 3 end of the nucleic acid linked to the anti-max antibody. This DNA polymerase is preferably the phi-29 polymerase known for its ability to efficiently replicate circular DNA.

### Example 3

### Interaction visualization enabled by the proximity of a nucleic acid carrying antibody and a DNA cleaving enzyme such as a restriction enzyme.

In this example the two proximity reagents are comprised of one antibody specific for myc carrying a nucleic acid capable of hybridizing to a circular RCA template and the other antibody specific for the max protein is linked to DNA restriction enzyme Hindlll. The nucleic acid of the first proximity probe is double stranded at the 3' end and when bound *in situ* and in proximity with the other proximity probe a proximity dependent cleavage event occurs where the Hindlll enzyme recognizes its cleavage site on the anti-myc probe resulting in a DNA polymerase accessible 3' end. This 3' end primes an RCA templated by the hybridized circular nucleic acid.

## Claims

1. A method for localised *in situ* detection of an analyte in a sample, comprising:
(a) contacting said sample with at least one set of at least first and second proximity probes, wherein said probes each comprise an analyte-binding moiety and can simultaneously bind to the analyte, and wherein
(i) said first proximity probe comprises a nucleic acid moiety attached at one end to the analyte-binding moiety, wherein a circular or circularisable oligonucleotide is hybridised to said nucleic acid moiety before, during or after said contacting step; and
(ii) said second proximity probe comprises an enzyme moiety, attached to the analyte-binding moiety, capable of directly or indirectly enabling rolling circle amplification (RCA) of the circular or, when it is circularised, of the circularisable oligonucleotide hybridised to the nucleic acid moiety of the first proximity probe, wherein said RCA is primed by said nucleic acid moiety of said first proximity probe;
(b) if necessary, circularising said oligonucleotide, to produce a circularised template for RCA;
(c) subjecting said circular or circularised template to RCA, wherein if the enzyme moiety of the second proximity probe in step (a)(ii) is a DNA polymerase, this step does not utilise a free DNA polymerase and wherein the nucleic acid moiety of the first proximity probe directly or indirectly mediates the priming of the RCA reaction; and
(d) detecting a product of said RCA.

2. The method of claim 1, wherein the nucleic acid moiety is attached at its 5' end to the analyte-binding moiety of the first proximity probe, and acts to prime the RCA reaction.

3. The method of claim 1, wherein the nucleic acid moiety is attached at its 3' end to the analyte-binding moiety of the first proximity probe and wherein a primer oligonucleotide is hybridised to, and overlaps the 5' end of, the nucleic acid moiety to provide a free 3' end which may act as the primer for the RCA reaction.

4. The method of any one of claims 1 to 3, wherein the analyte is a proteinaceous molecule, a nucleic acid molecule, a small organic or inorganic molecule, a cell, microorganism, or virus or a fragment or product thereof, a molecular complex, aggregate or a molecular interaction, or a modified form of a protein.

5. The method of any one of claims 1 to 4, wherein the sample is a cell or tissue sample.

6. The method of any one of claims 1 to 5, wherein a plurality of analytes is detected.

7. The method of any one of claims 1 to 6, wherein the analyte-binding moiety of said probes binds to the analyte directly, or wherein the analyte-binding moiety of said probes binds to the analyte indirectly via an intermediary binding partner which itself binds directly to the analyte, and preferably wherein the analyte binding moiety, and optionally the intermediary binding partner, is an antibody or a binding fragment, derivative or mimetic thereof, or an aptamer.

8. The method of any one of claims 1 to 7, wherein the oligonucleotide hybridised to the nucleic acid moiety is a linear circularisable oligonucleotide and (i) the ends of said oligonucleotide hybridise immediately adjacent to one another to contiguous parts of said nucleic acid moiety, or (ii) the ends of said oligonucleotide hybridise to non-contiguous parts of said nucleic acid moiety leaving a gap therebetween, which gap is filled by a gap oligonucleotide or by extending the 3' end of said linear circularisable oligonucleotide.

9. The method of any one of claims 1 to 8, wherein the enzyme moiety of said second proximity probe is a polymerase which acts to perform said RCA reaction.

10. The method of any one of claims 1 to 8, wherein the enzyme moiety of said second proximity probe is a ligase and the oligonucleotide which is hybridised to the nucleic acid moiety of the first proximity probe is a linear circularisable nucleotide, said method comprising in step (b) the step of circularising said oligonucleotide using said ligase enzyme moiety.

11. The method of any one of claims 1, 2 or 4 to 8, wherein the nucleic acid moiety of the first proximity probe is attached at its 5' end to the analyte-binding moiety and contains a modification at or near its 3' end which blocks polymerase-mediated extension, and wherein the enzyme moiety of said second proximity probe is a nucleic acid cleaving enzyme capable of cleaving off said modification, thereby to allow polymerase-mediated extension of said 3' end of the nucleic acid moiety, said method comprising in step (c), the step of subjecting said circular or circularised template to an RCA reaction primed by said nucleic acid moiety of said first proximity probe.

12. The method of claim 11 comprising one or more of the following features:
(i) wherein the nucleic acid cleaving enzyme moiety is a restriction or other endonuclease, an exonuclease or a DNA glycosylase,
(ii) the modification is a terminal 2'O methyl RNA residue, a Locked Nucleic Acid residue, a PNA residue, a terminator group or an inverse 3' end,
(iii) the nucleic acid moiety is provided with a cleavage site for said nucleic acid cleaving enzyme.

13. The method of any one of claims 1 to 12, wherein the RCA product is detected using labelled detection probes which bind to the product or labelled nucleotides which are incorporated into the product during the RCA, and preferably wherein said RCA product is detected by microscopic visualisation, flow cytometry or by using a scanning instrument.

14. A kit for localised *in situ* detection of an analyte in a sample, said kit comprising:
a set of proximity probes for at least one analyte wherein said set comprises at least a first and a second proximity probe, said first and second probes both comprising an analyte-binding moiety capable of directly or indirectly binding said analyte, wherein said probes can simultaneously bind to the analyte and wherein
said first probe additionally comprises a nucleic acid moiety and said second probe additionally comprises an enzyme moiety, said nucleic acid and enzyme moieties being as defined in any one of claims 1 to 3 or 8 to 12,
and a circular or circularisable oligonucleotide comprising a portion capable of hybridisirig to the nucleic acid moiety of said first proximity probe, wherein said circularisable oligonucleotide is capable of being circularised when hybridised to the nucleic acid moiety of said first proximity probe,
optionally together with one or more of the following components;
(i) if the analyte-binding moieties of said first and second probes are indirect analyte-binding moieties, direct analyte-binding moieties for which said analyte-binding moieties of the first and second probes have binding specificity;
(ii) one or more gap oligonucleotides capable of hybridising to a portion of the nucleic acid moiety of said first proximity probe;
(iii) a labelled oligonucleotide hybridisation probe capable of hybridising to a portion of said circular or circularisable oligonucleotide, or to a portion of said one or more gap oligonucleotides;
(iv) a ligase;
(v) a polymerase.

## Patentansprüche

1. Verfahren zum lokalisierten Nachweis eines Analyten in einer Probe, umfassend:
(a) Kontaktieren der Probe mit mindestens einem Satz von mindestens ersten und zweiten Näherungssonden, wobei die Sonden jeweils einen analytenbindenden Anteil umfassen und sich gleichzeitig an den Analyten binden können, und wobei
(i) die erste Näherungssonde einen Nukleinsäureanteil umfasst, der an einem Ende an dem analytenbindenden Anteil befestigt ist, wobei ein zirkuläres oder zirkularisierbares Oligonukleotid mit dem Nukleinsäureanteil vor, während oder nach dem Kontaktierungsschritt hybridisiert ist;
und
(ii) die zweite Näherungssonde einen Enzymanteil umfasst, der, befestigt an dem analytenbindenden Anteil, fähig ist, direkt oder indirekt die Rolling Circle Amplification (RCA) des zirkulären oder, wenn es zirkularisiert ist, des zirkularisierbaren Oligonukleotids zu ermöglichen, das mit dem Nukleotidanteil der ersten Näherungssonde hybridisiert ist, wobei die RCA durch den Nukleinsäureanteil der ersten Näherungssonde vorbereitet wird.
(b) falls notwendig, Zirkularisieren des Oligonukleotids, um ein zirkularisiertes Template für RCA zu erzeugen;
(c) Einwirkenlassen von RCA auf das zirkuläre oder zirkularisierte Template, wobei, falls der Enzymanteil der zweiten Näherungssonde in Schritt (a)(II) eine DNA-Polymerase ist, dieser Schritt keine freie DNA-Polymerase nutzt, und wobei der Nukleinsäureanteil der ersten Näherungssonde direkt oder indirekt das Vorbereiten (Priming) der RCA-Reaktion vermittelt; und
(d) Feststellen eines Produktes der RCA.

2. Verfahren nach Anspruch 1, wobei der Nukleinsäureanteil mit seinem 5'-Ende am analytenbindenden Anteil der ersten Näherungssonde befestigt ist und das Vorbereiten der RCA-Reaktion bewirkt.

3. Verfahren nach Anspruch 1, wobei der Nukleinsäureanteil mit seinem 3'-Ende am analytenbindenden Anteil der ersten Näherungssonde befestigt ist und wobei ein Primer-Oligonukleotid mit dem 5'-Ende des Nukleinsäureanteils hybridisiert ist und dasselbe überlappt, um für ein freies 3'-Ende zu sorgen, das als Primer für die RCA-Reaktion wirken kann.

4. Verfahren nach einem der Ansprüche 1 bis 3, wobei der Analyt ein proteinartiges Molekül, ein Nukleinsäuremolekül, ein kleines organisches oder anorganisches Molekül, eine Zelle, Mikroorganismus oder Virus oder ein Fragment oder Produkt desselben, ein molekularer Komplex, Aggregat oder eine molekulare Wechselwirkung oder eine modifizierte Form eines Proteins ist.

5. Verfahren nach einem der Ansprüche 1 bis 4, wobei die Probe eine Zell- oder Gewebsprobe ist.

6. Verfahren nach einem der Ansprüche 1 bis 5, wobei mehrere Analyten festgestellt werden.

7. Verfahren nach einem der Ansprüche 1 bis 6, wobei der analytenbindende Anteil der Sonden sich an den Analyten direkt bindet oder wobei der analytenbindende Anteil der Sonden sich indirekt über einen Zwischenbindungspartner bindet, der sich selbst direkt an den Analyten bindet, und wobei vorzugsweise der analytenbindende Anteil und optional der Zwischenbindungspartner ein Antikörper oder ein Bindungsfragment, -derivat oder -mimetikum desselben oder ein Aptamer ist.

8. Verfahren nach einem der Ansprüche 1 bis 7, wobei das Oligonukleotid, das mit dem Nukleinsäureanteil hybridisiert ist, ein lineares zirkularisierbares Oligonukleotid ist und (i) die Enden des Oligonukleotids unmittelbar aneinander angrenzend mit zusammenhängenden Teilen des Nukleinsäureanteils hybridisieren oder (ii) die Enden des Oligonukleotids mit nicht-zusammenhängenden Teilen des Nukleinsäureanteils hybridisieren, wobei ein Spalt dazwischen gelassen wird, welcher durch ein Spalten-Oligonukleotid gefüllt wird oder durch Erweitern des 3'-Endes des linearen zirkularisierbaren Oligonukleotids.

9. Verfahren nach einem der Ansprüche 1 bis 8, wobei der Enzymanteil der zweiten Näherungssonde eine Polymerase ist, die so wirkt, dass die RCA-Reaktion ausgeführt wird.

10. Verfahren nach einem der Ansprüche 1 bis 8, wobei der Enzymanteil der zweiten Näherungssonde eine Ligase ist, und das Oligonukleotid, das mit dem Nukleinsäureanteil der ersten Näherungssonde hybridisiert ist, ein lineares zirkularisierbares Nukleotid ist, wobei das Verfahren in Schritt (b) den Schritt des Zirkularisierens des Oligonukleotids unter Verwendung des Ligase-Enzymanteils umfasst.

11. Verfahren nach einem der Ansprüche 1, 2 oder 4 bis 8, wobei der Nukleinsäureanteil der ersten Näherungssonde mit ihrem 5'-Ende an dem analytenbindenden Anteil befestigt ist und eine Modifikation an ihrem 3'-Ende oder in der Nähe desselben enthält, die die polymerasevermittelte Erweiterung blockiert, und wobei der Enzymanteil der zweiten Näherungssonde ein nukleinsäurespaltendes Enzym ist, welches die Modifikation abspalten kann, wodurch die polymerasevermittelte Erweiterung des 3'-Endes des Nukleinsäureanteils ermöglicht wird, wobei das Verfahren in Schritt (c) den Schritt des Einwirkens einer RCA-Reaktion, die durch den Nukleinsäureanteil der ersten Näherungssonde geprimt wurde, auf zirkuläres oder zirkularisiertes Template umfasst.

12. Verfahren nach Anspruch 11, das ein oder mehrere der folgenden Merkmale umfasst:
(i) Der nukleinsäurespaltende Enzymanteil ist eine Restriktions- oder andere Endonuklease, eine Exonuklease oder eine DNA-Glycosylase,
(ii) Die Modifikation ist ein terminaler 2'O-Methyl-RNA-Rest, ein Locked-Nukleinsäure-Rest, ein PNA-Rest, eine Terminatorgruppe oder ein inverses 3'-Ende.
(iii) Der Nukleinsäureanteil ist mit einer Spaltungsstelle für das nukleinsäurespaltende Enzyme versehen.

13. Verfahren nach einem der Ansprüche 1 bis 12, wobei das RCA-Produkt unter Verwendung markierter Detektionssonden festgestellt wird, die sich an das Produkt oder markierte Nukleotide binden, welche während der RCA in das Produkt eingebaut werden, und wobei vorzugsweise das RCA-Produkt durch mikroskopische Sichtbarmachung, Durchflusszytometrie oder durch Verwendung eines Bildabtastungsinstrumentes festgestellt wird.

14. Baukasten zum lokalisierten Nachweis eines Analyten in einer Probe, umfassend:
einen Satz von Näherungssonden für mindestens einen Analyten, wobei der Satz mindestens eine erste und eine zweite Näherungssonde umfasst, wobei die erste und zweite Sonde jeweils einen analytenbindenden Anteil umfasst, der in der Lage ist, direkt oder indirekt den Analyten zu binden, wobei die Sonden sich gleichzeitig an den Analyten zu binden können und wobei die erste Sonde zusätzlich einen Nukleinsäureanteil umfasst und die zweite Sonde zusätzlich einen Enzymanteil umfasst, wobei die Nukleinsäure- und Enzymanteile wie in einem der Ansprüche 1 bis 3 oder 8 bis 12 definiert sind,
und ein zirkuläres oder zirkularisierbares Oligonukleotid, das einen Teil umfasst, der mit dem Nukleinsäureanteil der ersten Näherungssonde hybridisieren kann, wobei das zirkularisierbare Oligonukleotid zirkularisiert werden kann, wenn es mit dem Nukleinsäureanteil der ersten Näherungssonde hybridisiert ist,
optional zusammen mit einer oder mehreren der folgenden Komponenten;
(i) wenn die analytenbindenden Anteile der ersten und zweiten Sonde indirekte analytenbindende Anteile sind, direkte analytenbindende Anteile, für welche die analytenbindenden Anteile der ersten und zweiten Sonde eine Bindungsspezifität haben;
(ii) ein oder mehrere Spalten-Oligonukleotide, die mit einem Teil des Nukleinsäureanteils der ersten Näherungssonde hybridisieren können;
(iii) eine markierte Oligonukleotid-Hybridisierungssonde, die mit einem Teil des zirkulären oder zirkularisierbaren Oligonukleotids oder mit einem Teil des einen oder der mehreren Oligonukleotide hybridisieren kann;
(iv) eine Ligase;
(v) eine Polymerase.

## Revendications

1. Procédé pour la détection localisée *in situ* d'un analyte dans un échantillon, comprenant les étapes consistant à :
(a) mettre en contact ledit échantillon avec au moins un ensemble d'au moins une première et une seconde sonde de proximité, dans lequel lesdites sondes comprennent chacune une fraction se liant à l'analyte et peuvent simultanément se lier à l'analyte, et dans lequel :
(i) ladite première sonde de proximité comprend une fraction d'acide nucléique fixée, à une terminaison, à la fraction se liant à l'analyte, dans lequel un oligonucléotide circulaire ou circularisable est hybridé à ladite fraction d'acide nucléique avant, pendant ou après ladite étape de mise en contact ; et
(ii) ladite seconde sonde de proximité comprend une fraction d'enzyme fixée à la fraction se liant à l'analyte et capable de permettre directement ou indirectement une amplification par cercle roulant (RCA) de l'oligonucléotide circulaire ou, lorsqu'il est circularisé, de l'oligonucléotide circularisable hybridé à la fraction d'acide nucléique de la première sonde de proximité, dans lequel la RCA est amorcée par ladite fraction d'acide nucléique de ladite première sonde de proximité ;
(b) si nécessaire, circulariser ledit oligonucléotide pour produire une matrice circularisée pour la RCA ;
(c) soumettre ladite matrice circulaire ou circularisée à une RCA, dans lequel, si la fraction d'enzyme de la seconde sonde de proximité à l'étape (a)(ii) est une polymérase d'ADN, cette étape n'utilise pas une polymérase d'ADN libre et dans lequel la fraction d'acide nucléique de la première sonde de proximité médie directement ou indirectement l'amorçage de la réaction de RCA ; et
(d) détecter un produit de ladite RCA.

2. Procédé selon la revendication 1, dans lequel la fraction d'acide nucléique est fixée, à sa terminaison 5', à la fraction se liant à l'analyte de la première sonde de proximité et agit pour amorcer la réaction de RCA.

3. Procédé selon la revendication 1, dans lequel la fraction d'acide nucléique est fixée, à sa terminaison 3', à la fraction se liant à l'analyte de la première sonde de proximité et dans lequel un oligonucléotide amorce est hybridé à la fraction d'acide nucléique et chevauche la terminaison 5' de celle-ci pour fournir une terminaison 3' libre qui peut agir comme amorce pour la réaction de RCA.

4. Procédé selon l'une quelconque des revendications 1 à 3, dans lequel l'analyte est une molécule protéinée, une molécule d'acide nucléique, une petite molécule organique ou inorganique, une cellule, un micro-organisme ou un virus ou un fragment ou un produit de celui-ci, un complexe moléculaire, un agrégat ou une interaction moléculaire ou encore une forme modifiée d'une protéine.

5. Procédé selon l'une quelconque des revendications 1 à 4, dans lequel l'échantillon est un échantillon de cellule ou de tissu.

6. Procédé selon l'une quelconque des revendications 1 à 5, dans lequel on détecte une pluralité d'analytes.

7. Procédé selon l'une quelconque des revendications 1 à 6, dans lequel la fraction se liant à l'analyte desdites sondes se lie à l'analyte directement ou dans lequel la fraction se liant à l'analyte desdites sondes se lie à l'analyte indirectement via un partenaire de liaison intermédiaire qui se lie lui-même directement à l'analyte et, de préférence, dans lequel la fraction se liant à l'analyte et, éventuellement, le partenaire de liaison intermédiaire sont un anticorps ou un fragment de liaison, un dérivé ou un mimétique de ceux-ci ou encore un aptamère.

8. Procédé selon l'une quelconque des revendications 1 à 7, dans lequel l'oligonucléotide hybridé à la fraction d'acide nucléique est un oligonucléotide linéaire circularisable et (i) les terminaisons dudit oligonucléotide s'hybrident immédiatement au voisinage l'une de l'autre à des parties contiguës de ladite fraction d'acide nucléique ou (ii) les terminaisons dudit oligonucléotide s'hybrident à des parties non contiguës de ladite fraction d'acide nucléique en laissant une brèche entre elles, laquelle brèche est remplie par un oligonucléotide de brèche ou en étendant la terminaison 3' dudit oligonucléotide linéaire circularisable.

9. Procédé selon l'une quelconque des revendications 1 à 8, dans lequel la fraction d'enzyme de ladite seconde sonde de proximité est une polymérase qui agit pour effectuer ladite réaction de RCA.

10. Procédé selon l'une quelconque des revendications 1 à 8, dans lequel la fraction d'enzyme de ladite seconde sonde de proximité est une ligase et l'oligonucléotide qui est hybridé à la fraction d'acide nucléique de la première sonde de proximité est un nucléotide linéaire circularisable, ledit procédé comprenant à l'étape (b) l'étape de circularisation dudit oligonucléotide en utilisant ladite fraction d'enzyme ligase.

11. Procédé selon l'une quelconque des revendications 1, 2 ou 4 à 8, dans lequel la fraction d'acide nucléique de la première sonde de proximité est fixée, à sa terminaison 5', à la fraction se liant à l'analyte et contient une modification à sa terminaison 3' ou à proximité de celle-ci, qui bloque une extension médiée par polymérase, et dans lequel la fraction d'enzyme de ladite seconde sonde de proximité est un enzyme de clivage d'acide nucléique capable de cliver ladite modification de manière à permettre l'extension médiée par polymérase de ladite terminaison 3' de la fraction d'acide nucléique, ledit procédé comprenant à l'étape (c) l'étape de soumission de ladite matrice circulaire ou circularisée à une réaction de RCA amorcée par ladite fraction d'acide nucléique de ladite première sonde de proximité.

12. Procédé selon la revendication 11, comprenant une ou plusieurs des caractéristiques suivantes :
(i) la fraction d'enzyme de clivage d'acide nucléique est un enzyme de restriction ou une autre endonucléase, une exonucléase ou une glycosylase d'ADN,
(ii) la modification est un résidu d'ARN 2'-O-méthyle, un résidu d'acide nucléique bloqué, un résidu PNA, un groupe terminateur ou une terminaison 3' inverse,
(iii) la fraction d'acide nucléique est pourvue d'un site de clivage pour ledit enzyme de clivage d'acide nucléique.

13. Procédé selon l'une quelconque des revendications 1 à 12, dans lequel le produit de RCA est détecté en utilisant des sondes de détection marquées qui se lient au produit ou des nucléotides marqués qui sont incorporés au produit au cours de la RCA et, de préférence, dans lequel ledit produit de RCA est détecté par visualisation au microscope, cytométrie en flux ou en utilisant un instrument de balayage.

14. Kit pour détection localisée *in situ* d'un analyte
dans un échantillon, ledit kit comprenant :
un ensemble de sondes de proximité pour au moins un analyte, dans lequel ledit ensemble comprend au moins une première et une seconde sonde de proximité, lesdites première et seconde sondes de proximité comprenant toutes deux une fraction se liant à l'analyte capable de se lier directement ou indirectement audit analyte, dans lequel lesdites sondes peuvent simultanément se lier à l'analyte et dans lequel ladite première sonde comprend en outre une fraction d'acide nucléique et ladite seconde sonde comprend en outre une fraction d'enzyme, lesdites fractions d'acide nucléique et d'enzyme étant définies dans l'une quelconque des revendications 1 à 3 ou 8 à 12, et
un oligonucléotide circulaire ou circularisable
comprenant une portion capable de s'hybrider à la fraction d'acide nucléique de ladite première sonde de proximité, dans lequel ledit oligonucléotide circularisable est capable de se circulariser lorsqu'il est hybridé à la fraction d'acide nucléique de ladite première sonde de proximité,
éventuellement conjointement avec un ou plusieurs des
composants suivants :
(i) si les fractions se liant à l'analyte desdites première et seconde sondes sont des fractions se liant indirectement à l'analyte, des fractions se liant directement à l'analyte pour lesquelles lesdites factions se liant à l'analyte des première et seconde sondes ont une spécificité de liaison ;
(ii) un ou plusieurs oligonucléotides de brèche capables de s'hybrider à une portion de la fraction d'acide nucléique de ladite première sonde de proximité ;
(iii) une sonde d'hybridation d'oligonucléotide marquée capable de s'hybrider à une portion dudit oligonucléotide circulaire ou circularisable ou à une portion desdits un ou plusieurs oligonucléotides de brèche ;
(iv) une ligase ; et
(v) une polymérase.
